# EUROPÄISCHE PATENTSCHRIFT

(11) **EP 1 534 713 B1**
(45) Veröffentlichungstag und Bekanntmachung des Hinweises auf die Patenterteilung: **11.01.2006**
(21) Anmeldenummer: 03797239.5
(22) Anmeldetag: 13.08.2003
(51) Int. Cl.: C07D 487/04, A61K 31/519, A61P 25/28

(54) **ALKYL-SUBSTITUIERTE PYRAZOLOPYRIMIDINE**
ALKYL-SUBSTITUTED PYRAZOLOPYRIMIDINES
PYRAZOLOPYRIMIDINES SUBSTITUEES PAR ALKYLE

(30) Priorität: 23.08.2002 DE 10238724
(43) Veröffentlichungstag der Anmeldung: 01.06.2005
(73) Patentinhaber: Bayer HealthCare AG, 51368 Leverkusen (DE)
(72) Erfinder: HENDRIX, Martin, 51519 Odenthal (DE); BÖSS, Frank-Gerhard, Berkshire SL5 7DF (GB); BURKHARDT, Nils, 42553 Velbert (DE); ERB, Christina, 65830 Kriftel (DE); TERSTEEGEN, Adrian, 42553 Velbert (DE); VAN KAMPEN, Marja, 40223 Düsseldorf (DE)
(86) Internationale Anmeldenummer: PCT/EP2003/008979
(87) Internationale Veröffentlichungsnummer: WO 2004/026876

(56) Entgegenhaltungen:
- EP-A- 0 995 751
- WO-A-02/055082
- WO-A-03/037899
- CH-A- 396 925
- DE-B- 1 156 415
- US-A- 5 977 118

## Beschreibung

Die Erfindung betrifft neue Alkyl-substituierte Pyrazolopyrimidine, Verfahren zu ihrer Herstellung, und ihre Verwendung zur Herstellung von Arzneimitteln zur Verbesserung von Wahrnehmung, Konzentrationsleistung, Lern- und/oder Gedächtnisleistung.

Die zelluläre Aktivierung von Adenylat- bzw. Guanylatzyklasen bewirkt die Zyklisierung von ATP bzw. GTP zu 5'-3' zyklischem Adenosin-Monophosphat (cAMP) bzw. 5'-3' zyklischem Guanosin-Monophosphat (cGMP). Diese zyklischen Nukleotide (cAMP und cGMP) sind wichtige second messenger und spielen daher eine zentrale Rolle in den zellulären Signaltransduktionskaskaden. Beide aktivieren unter anderem, aber nicht ausschließlich, jeweils wieder Protein-Kinasen. Die von cAMP aktivierte Protein-Kinase wird Protein-Kinase A (PKA) genannt, die von cGMP aktivierte Protein-Kinase wird Protein-Kinase G (PKG) genannt. Aktivierte PKA bzw. PKG können wiederum eine Reihe zellulärer Effektorproteine phosphorylieren (z.B. Ionenkanäle, G-Protein gekoppelte Rezeptoren, Strukturproteine). Auf diese Weise können die second messengers cAMP und cGMP die unterschiedlichsten physiologischen Vorgänge in den verschiedensten Organen kontrollieren. Die zyklischen Nukleotide können aber auch direkt auf Effektormoleküle wirken. So ist z.B. bekannt, dass cGMP direkt auf Ionenkanäle wirken kann und hiermit die zelluläre Ionenkonzentration beeinflussen kann (Übersicht in: Wei et al., *Prog. Neurobiol.,* 1998, *56*: 37-64). Ein Kontrollmechanismus, um die Aktivität von cAMP und cGMP und damit diese physiologischen Vorgänge wiederum zu steuern, sind die Phosphodiesterasen (PDE). PDEs hydrolysieren die zyklischen Monophosphate zu den inaktiven Monophosphaten AMP und GMP. Es sind mittlerweile mindestens 21 PDE-Gene beschrieben (*Exp. Opin. Investig. Drugs* 2000, *9,* 1354-3784). Diese 21 PDE-Gene lassen sich aufgrund ihrer Sequenzhomologie in 11 PDE-Familien einteilen (Nomenklatur Vorschlag siehe http://depts.washington.edu/pde/Nomenclature.html.). Einzelne PDE-Gene innerhalb einer Familie werden durch Buchstaben unterschieden (z.B. PDE1A und PDE1B). Falls noch unterschiedliche Splice Varianten innerhalb eines Genes vorkommen, wird dies dann durch eine zusätzliche Nummerierung nach dem Buchstaben angegeben (z.B. PDE1A1).

Die humane PDE9A wurde 1998 kloniert und sequenziert. Die Aminosäurenidentität zu anderen PDEs liegt bei maximal 34 % (PDE8A) und minimal 28 % (PDE5A). Mit einer Michaelis-Menten-Konstante (Km-Wert) von 170 nM ist PDE9A hochaffin für cGMP. Darüber hinaus ist PDE9A selektiv für cGMP (Km-Wert für cAMP = 230 µM). PDE9A weist keine cGMP Bindungsdomäne auf, die auf eine allosterische Enzymregulation durch cGMP schließen ließe. In einer Western-Blot-Analyse wurde gezeigt, daß die PDE9A im Mensch in Hoden, Gehirn, Dünndarm, Skelettmuskulatur, Herz, Lunge, Thymus und Milz exprimiert wird. Die höchste Expression wurde in Gehirn, Dünndarm, Herz und Milz gefunden (Fisher et al., *J. Biol. Chem*., 1998, *273* (25): 15559-15564). Das Gen für die humane PDE9A liegt auf Chromosom 21q22.3 und enthält 21 Exons. Bislang wurden 4 alternative Spleißvarianten der PDE9A identifiziert (Guipponi et al., *Hum. Genet*., 1998, *103*: 386-392). Klassische PDE-Inhibitoren hemmen die humane PDE9A nicht. So zeigen IBMX, Dipyridamole, SKF94120, Rolipram und Vinpocetin in Konzentrationen bis 100 µM keine Inhibition am isolierten Enzym. Für Zaprinast wurde ein IC₅₀-Wert von 35 µM nachgewiesen (Fisher et al., *J. Biol. Chem.,* 1998, *273* (25): 15559-15564).

Die Maus-PDE9A wurde 1998 von Soderling et al. (*J. Biol. Chem*., 1998, *273* (19): 15553-15558) kloniert und sequenziert. Diese ist wie die humane Form hochaffin für cGMP mit einem Km von 70 nM. In der Maus wurde eine besonders hohe Expression in der Niere, Gehirn, Lunge und Herz gefunden. Auch die Maus-PDE9A wird von IBMX in Konzentrationen unter 200 µM nicht gehemmt; der IC₅₀-Wert für Zaprinast liegt bei 29 µM (Soderling et al., *J. Biol. Chem.,* 1998, *273* (19): 15553-15558). Im Rattengehirn wurde gezeigt, daß PDE9A in einigen Hirnregionen stark exprimiert wird. Dazu zählen der Bulbus olfactorius, Hippocampus, Cortex, Basalganglien und basales Vorderhirn (Andreeva et al., *J. Neurosci*., 2001, *21* (22): 9068-9076). Insbesondere Hippokampus, Cortex und basales Vorderhirn spielen eine wichtige Rolle an Lern- und Gedächtnisvorgängen.

Wie oben bereits erwähnt, zeichnet sich PDE9A durch eine besonders hohe Affinität für cGMP aus. Deshalb ist PDE9A im Gegensatz zu PDE2A (Km = 10 µM; Martins et al., *J. Biol. Chem*., 1982, *257*: 1973-1979), PDE5A (Km = 4 µM; Francis et al., *J. Biol. Chem*., 1980, *255*: 620-626), PDE6A (Km = 17 µM; Gillespie and Beavo, *J*. *Biol. Chem*., 1988, *263* (17): 8133-8141) und PDE11A (Km = 0.52 µM; Fawcett et al., *Proc. Nat. Acad. Sci*., 2000, 97 (7): 3702-3707) schon bei niedrigen physiologischen Konzentrationen aktiv. Im Gegensatz zu PDE2A (Murashima et al., *Biochemistry*, 1990, *29*: 5285-5292) wird die katalytische Aktvität von PDE9A nicht durch cGMP gesteigert, da es keine GAF-Domäne (cGMP-Bindedomäne, über die die PDE-Aktivität allosterisch gesteigert wird) aufweist (Beavo et al., *Current Opinion in Cell Biology,* 2000, *12*: 174-179). PDE9A-Inhibitoren führen deshalb zu einer Erhöhung der basalen cGMP-Konzentration. Diese Erhöhung der basalen cGMP-Konzentration führte überraschenderweise zu einer Verbesserung der Lern- und Gedächtnisleistung im Social Recognition Test.

Die WO 98/40384 offenbart Pyrazolopyrimidine, die sich als PDE1-, 2- und 5-Inhibitoren auszeichnen und für die Behandlung von cardiovasculären und cerebrovasculären Erkrankungen sowie Erkrankungen des Urogenitalbereiches eingesetzt werden können. Weitere Pyrazolopyrimidine mit PDE-inhibierender Wirkung sind in US 5,977,118 and EP 0 995 751 offenbart.

In CH 396 924, CH 396 925, CH 396 926, CH 396 927, DE 1 147 234, DE 1 149 013, GB 937,726 werden Pyrazolopyrimidine mit coronarerweiternder Wirkung beschrieben, die zur Behandlung von Durchblutungsstörungen des Herzmuskels eingesetzt werden können.

Im US 3,732,225 werden Pyrazolopyrimidine beschrieben, die eine entzündungshemmende und Blutzucker-senkende Wirkung haben.

In DE 2 408 906 werden Styrolpyrazolpyrimidine beschrieben, die als antimikrobielle und entzündungshemmende Mittel für die Behandlung von beispielsweise Ödem eingesetzt werden können.

Die vorliegende Erfindung betrifft Verbindungen der Formel 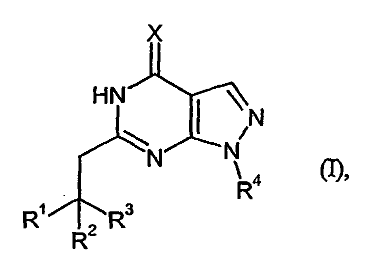
in welcher
- R¹: C₁-C₆-Alkyl, Trifluormethyl, Hydroxy, C₁-C₆-Alkoxy, -C(=O)OR⁵ oder -C(=O)NR⁶R⁷, wobei C₁-C₆-Alkyl gegebenenfalls mit 1 bis 3 Resten unabhängig voneinander ausgewählt aus der Gruppe Hydroxy, C₁-C₆-Alkoxy, Halogen, Trifluormethyl, Trifluormethoxy, -C(=O)OR⁵ oder -C(=O)NR⁶R⁷ substituiert ist, und
R⁵ für C₁-C₆-Alkyl,
R⁶ und R⁷ unabhängig voneinander für Wasserstoff, C₆-C₁₀-Aryl, C₁-C₆₋Alkyl stehen, oder
zusammen mit dem Stickstoffatom, an das sie gebunden sind, ein 4- bis 10-gliedriges Heterocyclyl bilden,
- R²: Wasserstoff, C₁-C₆-Alkyl, Trifluormethyl, C₁-C₆-Alkoxy,

oder
- R¹ und R²: zusammen mit dem Kohlenstoffatom, an das sie gebunden sind, C₃₋C₈-Cycloalkyl, C₃-C₈-Cycloalkenyl oder 4- bis 10-gliedriges Heterocyclyl bilden, die gegebenenfalls mit bis zu 2 Substituenten aus der Gruppe C₁-C₆-Alkyl, C₁-C₆-Alkoxy, Hydroxy, Oxo, -C(=O)OR⁸ substituiert sind, und
R⁸ für C₁-C₆-Alkyl oder Benzyl steht,
- R³: Wasserstoff oder C₁-C₆-Alkyl,
- R⁴: Pentan-3-yl, C₃-C₆-Cycloalkyl,
- X: Sauerstoff oder Schwefel,
bedeuten, sowie deren Salze, Solvate und/oder Solvate der Salze.

Erfindungsgemäße Verbindungen sind die Verbindungen der Formel (I) und deren Salze, Solvate und Solvate der Salze; die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Als Salze sind im Rahmen der Erfindung physiologisch unbedenkliche Salze der erfindungsgemäßen Verbindungen bevorzugt.

Physiologisch unbedenkliche Salze der Verbindungen (I) umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure und Benzoesäure.

Physiologisch unbedenkliche Salze der Verbindungen (I) umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methyhnorpholin, Dehydroabietylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Außerdem betrifft die vorliegende Offenbarung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
C₁-C₆-Alkoxy steht für einen geradkettigen oder verzweigten Alkoxyrest mit 1 bis 6, bevorzugt 1 bis 4, besonders bevorzugt mit 1 bis 3 Kohlenstoffatomen. Nicht-limitierende Beispiele umfassen Methoxy, Ethoxy, n-Propoxy, Isopropoxy, tert.-Butoxy, n-Pentoxy und n-Hexoxy.
C₁-C₆-Alkyl steht für einen geradkettigen oder verzweigten Alkylrest mit 1 bis 6, bevorzugt 1 bis 4, besonders bevorzugt 1 bis 3 Kohlenstoffatomen. Nicht-limitierende Beispiele umfassen Methyl, Ethyl, n-Propyl, Isopropyl, tert.-Butyl, n-Pentyl und n-Hexyl.
C₆-C₁₀-Aryl steht für Phenyl oder Naphthyl.
C₃-C₈-Cycloalkyl steht für Cyclopropyl, Cyclopentyl, Cyclobutyl, Cyclohexyl, Cycloheptyl oder Cyclooctyl. Bevorzugt seien Cyclopropyl, Cyclopentyl und Cyclohexyl genannt.
C₃-C₈-Cycloalkenyl steht für teilweise ungesättigte, nicht-aromatische Cycloalkylreste, die eine oder mehrere Mehrfachbindungen, vorzugsweise Doppelbindungen enthalten.. Nicht-limitierende Beispiele umfassen Cyclopentenyl, Cyclohexenyl und Cycloheptenyl.
Halogen steht für Fluor, Chlor, Brom und Iod. Bevorzugt sind Fluor, Chlor, Brom, besonders bevorzugt Fluor und Chlor.
4- bis 10-gliedriges Heterocyclyl steht für einen mono- oder polycyclischen, heterocyclischen Rest mit 4 bis 10 Ringatomen und bis zu 3, vorzugsweise 1 Heteroatomen bzw. Heterogruppen aus der Reihe N, O, S, SO, SO₂. 4- bis 8-gliedriges Heterocyclyl ist bevorzugt. Mono- oder bicyclisches Heterocyclyl ist bevorzugt. Als Heteroatome sind N und O bevorzugt. Die Heterocyclyl-Reste können gesättigt oder teilweise ungesättigt sein. Gesättigte Heterocyclyl-Reste sind bevorzugt. Die Heterocyclyl-Reste können über ein Kohlenstoffatom oder ein Heteroatom gebunden sein. Besonders bevorzugt sind 5- bis 7-gliedrige, monocyclische gesättigte Heterocyclylreste mit bis zu zwei Heteroatomen aus der Reihe O, N und S. Beispielsweise und vorzugsweise seien genannt: Oxetan-3-yl, Pyrrolidin-2-yl, Pyrrolidin-3-yl, Pyrrolinyl, Tetrahydrofuranyl, Tetrahydrothienyl, Pyranyl, Piperidinyl, Thiopyranyl, Morpholinyl, Perhydroazepinyl.

Wenn Reste in den erfindungsgemäßen Verbindungen gegebenenfalls substituiert sind, ist, soweit nicht anders spezifiziert, eine Substitution mit bis zu drei gleichen oder verschiedenen Substituenten bevorzugt.

Die erfindungsgemäßen Verbindungen können auch als Tautomere vorliegen, wie im Folgenden beispielhaft gezeigt wird:

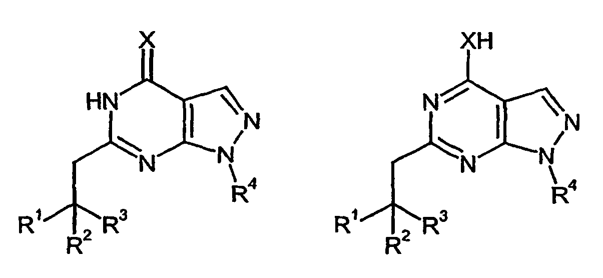

Eine weitere Ausführungsform der Erfindung betrifft Verbindungen der Formel (I), wobei
- R¹: C₁-C₆-Alkyl, Hydroxy, C₁-C₆-Alkoxy, -C(=O)OR⁵ oder -C(=O)NR⁶R⁷, wobei C₁-C₆-Alkyl gegebenenfalls mit Hydroxy, C₁-C₆-Alkoxy, -C(=O)OR⁵ oder -C(=O)NR⁶R⁷ substituiert ist, und
R⁵ für C₁-C₆-Alkyl,
R⁶ und R⁷ unabhängig voneinander für Wasserstoff, C₆-C₁₀-Aryl, C₁-C₆₋Alkyl stehen, oder
zusammen mit dem Stickstoffatom, an das sie gebunden sind, ein 4- bis 10-gliedriges Heterocyclyl bilden,
- R²: Wasserstoff, C₁-C₆-Alkyl, C₁-C₆-Alkoxy,

oder
- R¹ und R²: zusammen mit dem Kohlenstoffatom, an das sie gebunden sind, C₃₋C₈-Cycloalkyl, C₃-C₈-Cycloalkenyl oder 4- bis 10-gliedriges Heterocyclyl bilden, die gegebenenfalls mit bis zu 2 Substituenten aus der Gruppe C₁-C₆-Alkyl, C₁-C₆-Alkoxy, Hydroxy, Oxo, -C(=O)OR⁸ substituiert sind, und
R⁸ für C₁-C₆-Alkyl oder Benzyl steht,
- R³: Wasserstoff oder C₁-C₆-Alkyl,
- R⁴: Pentan-3-yl, C₄-C₆-Cycloalkyl,
- X: Sauerstoff oder Schwefel,
bedeuten, sowie deren Salze, Solvate und/oder Solvate der Salze.

Eine weitere Ausführungsform der Erfindung betrifft Verbindungen der Formel (I), wobei
- R¹: C₁-C₄-Alkyl, Trifluormethyl, Hydroxy, C₁-C₄-Alkoxy, -C(=O)OR⁵ oder -C(=O)NR⁶R⁷, wobei C₁-C₄-Alkyl gegebenenfalls mit Hydroxy, C₁-C₄₋Alkoxy, Trifluormethyl, -C(=O)OR⁵ oder -C(=O)NR⁶R⁷ substituiert ist, und
R⁵ für C₁-C₄-Alkyl,
R⁶ und R⁷ unabhängig voneinander für Wasserstoff, Phenyl, C₁-C₄-Alkyl stehen, oder
zusammen mit dem Stickstoffatom, an das sie gebunden sind, ein 5- bis 6-gliedriges Heterocyclyl bilden,
- R²: Wasserstoff, C₁-C₄-Alkyl, C₁-C₄-Alkoxy, Trifluormethyl,

oder
- R¹ und R²: zusammen mit dem Kohlenstoffatom, an das sie gebunden sind, C₅₋C₆-Cycloalkyl, C₅-C₆-Cycloalkenyl oder 5- bis 6-gliedriges Heterocyclyl bilden, die gegebenenfalls mit bis zu 2 Substituenten aus der Gruppe C₁-C₄-Alkyl, C₁-C₄-Alkoxy, Hydroxy, Oxo, -C(=O)OR⁸ substituiert sind, und
- R⁸: für C₁-C₄-Alkyl oder Benzyl steht,
- R³: Wasserstoff,
- R⁴: Pentan-3-yl, C₅-C₆-Cycloalkyl,
- X: Sauerstoff oder Schwefel,
bedeuten, sowie deren Salze, Solvate und/oder Solvate der Salze.

Eine weitere Ausführungsform der Erfindung betrifft Verbindungen der Formel (I), wobei
- R¹: Methyl, Ethyl, Isopropyl, Trifluormethyl, Methoxycarbonyl, Ethoxycarbonyl oder -C(=O)NR⁶R⁷, wobei Methyl gegebenenfalls mit Methoxycarbonyl oder Ethoxycarbonyl substituiert ist, und
R⁶ für Phenyl steht und
R⁷ für Wasserstoff steht,
- R²: Wasserstoff, Methyl, Trifluormethyl, oder
- R¹ und R²: zusammen mit dem Kohlenstoffatom, an das sie gebunden sind, Cyclopentyl, Cyclohexyl, Cyclopentenyl oder Tetrahydrofuryl bilden, wobei Cyclohexyl gegebenenfalls mit Methyl substituiert ist, und
- R³: Wasserstoff,
- R⁴: Pentan-3-yl, C₅-C₆-Cycloalkyl,
- X: Sauerstoff oder Schwefel,
bedeuten, sowie deren Salze, Solvate und/oder Solvate der Salze.

Eine weitere Ausführungsform der Erfindung betrifft Verbindungen der Formel (I), wobei
- R¹: Methyl, Ethyl, Isopropyl, Methoxycarbonyl, Ethoxycarbonyl oder -C(=O)NR⁶R⁷, wobei Methyl gegebenenfalls mit Methoxycarbonyl oder Ethoxycarbonyl substituiert ist, und
R⁶ für Phenyl steht und
R⁷ für Wasserstoff stehen,
- R²: Wasserstoff, Methyl, oder
- R¹ und R²: zusammen mit dem Kohlenstoffatom, an das sie gebunden sind, Cyclopentyl, Cyclohexyl, Cyclopentenyl oder Tetrahydrofuryl bilden, wobei Cyclohexyl gegebenenfalls mit Methyl substituiert ist, und
- R³: Wasserstoff,
- R⁴: Pentan-3-yl, C₅-C₆-Cycloalkyl,
- X: Sauerstoff,
bedeuten, sowie deren Salze, Solvate und/oder Solvate der Salze.

Außerdem wurde ein Verfahren zur Herstellung der erfindungsgemäßen Verbindungen der Formel (I) gefunden, dadurch gekennzeichnet, dass man entweder
[A] Verbindungen der Formel 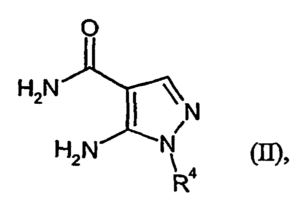 in welcher R⁴ die oben angegebenen Bedeutungen hat, durch Umsetzung mit einer Verbindung der Formel 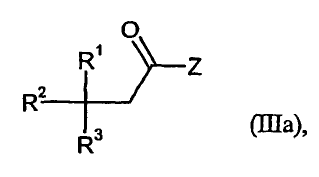
   in welcher R¹, R² und R³ die oben angegebenen Bedeutungen haben,
   und
   - Z: für Chlor oder Brom steht,

   in einem inerten Lösemittel und in Anwesenheit einer Base zunächst in Verbindungen der Formel 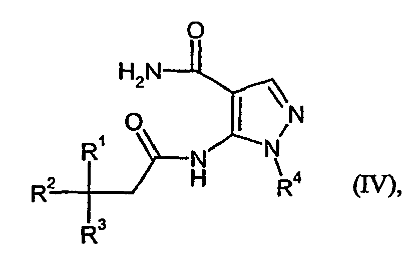
   in welcher R¹, R², R³ und R⁴ die oben angegebenen Bedeutungen haben,
   überführt, dann in einem inerten Lösemittel in Gegenwart einer Base zu Verbindungen der Formel 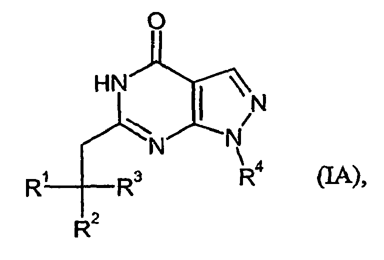
   in welcher R¹, R², R³ und R⁴ die oben angegebenen Bedeutungen haben,
   cyclisiert,
   oder
[B] Verbindungen der Formel (II) unter direkter Cyclisierung zu (IA) mit einer Verbindung der Formel 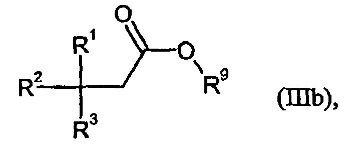
   in welcher R¹, R² und R³ die oben angegebenen Bedeutungen haben
   und
   - R⁹: für Methyl oder Ethyl steht,

   in einem inerten Lösemittel und in Anwesenheit einer Base umsetzt,
   oder
[C] Verbindungen der Formel 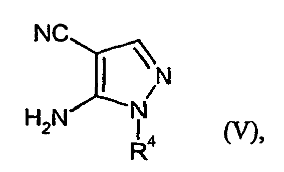
   in welcher R⁴ die oben angegebenen Bedeutungen hat,
   zunächst durch Umsetzung mit einer Verbindung der Formel (IIIa) in einem inerten Lösemittel und in Anwesenheit einer Base in Verbindungen der Formel 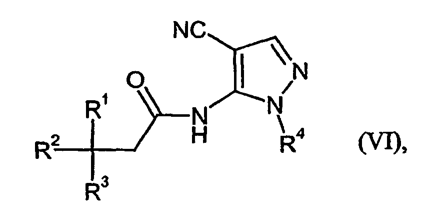
   in welcher R¹, R², R³ und R⁴ die oben angegebenen Bedeutungen haben,
   überführt,
   und diese in einem zweiten Schritt in einem inerten Lösemittel und in Anwesenheit einer Base und eines Oxidationsmittels zu (IA) cyclisiert,

und die Verbindungen der Formel (IA) gegebenenfalls dann durch Umsetzung mit einem Schwefelungsmittel wie beispielsweise Diphosphorpentasulfid in die Thiono-Derivate der Formel 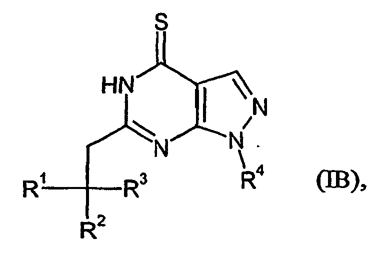
in welcher R¹, R², R³ und R⁴ die oben angegebenen Bedeutungen haben,
überführt,
und die resultierenden Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

Für den ersten Schritt des Verfahrens [A] und des Verfahrens [C] eignen sich inerte organische Lösemittel, die sich unter den Reaktionsbedingungen nicht verändern. Hierzu gehören bevorzugt Ether wie beispielsweise Diethylether, Dioxan, Tetrahydrofuran oder Glykoldimethylether, oder Toluol oder Pyridin. Ebenso ist es möglich, Gemische der genannten Lösemittel einzusetzen. Besonders bevorzugt sind Tetrahydrofuran, Toluol oder Pyridin.

Als Basen eignen sich im allgemeinen Alkalihydride, wie beispielsweise Natriumhydrid, oder cyclische Amine, wie beispielsweise Piperidin, Pyridin, Dimethylaminopyridin (DMAP), oder C₁-C₄-Alkylamine, wie beispielsweise Triethylamin. Bevorzugt sind Natriumhydrid, Pyridin und/oder Dimethylaminopyridin.

Die Base wird im allgemeinen in einer Menge von 1 mol bis 4 mol, bevorzugt von 1.2 mol bis 3 mol, jeweils bezogen auf 1 mol der Verbindungen der allgemeinen Formel (II) bzw. (V), eingesetzt.

In einer Variante wird die Umsetzung in Pyridin, dem eine katalytische Menge DMAP zugesetzt wird, durchgeführt. Gegebenenfalls kann noch Toluol zugefügt werden.

Die Reaktionstemperatur kann im allgemeinen in einem größeren Bereich variiert werden. Im allgemeinen arbeitet man in einem Bereich von -20°C bis +200°C, bevorzugt von 0°C bis +100°C.

Als Lösemittel für die Cyclisierung im zweiten Schritt der Verfahren [A] und [C] eignen sich die üblichen organischen Lösemittel. Hierzu gehören bevorzugt Alkohole wie Methanol, Ethanol, Propanol, Isopropanol, n-Butanol oder tert.-Butanol, oder Ether wie Tetrahydrofuran oder Dioxan, oder Dimethylformamid oder Dimethylsulfoxid. Besonders bevorzugt werden Alkohole wie Methanol, Ethanol, Propanol, Isopropanol oder tert.-Butanol verwendet. Ebenso ist es möglich, Gemische der genannten Lösemittel einzusetzen.

Als Basen für die Cyclisierung im zweiten Schritt der Verfahren [A] und [C] eignen sich die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.-butanolat. Besonders bevorzugt sind Kaliumcarbonat, Natriumhydroxid und Kalium-tert.-butanolat.

Bei der Durchführung der Cyclisierung wird die Base im allgemeinen in einer Menge von 2 mol bis 6 mol, bevorzugt von 3 mol bis 5 mol, jeweils bezogen auf 1 mol der Verbindungen der allgemeinen Formel (IV) bzw. (VI), eingesetzt.

Als Oxidationsmittel für die Cyclisierung im zweiten Schritt des Verfahrens [C] eignen sich beispielsweise Wasserstoffperoxid oder Natriumborat. Bevorzugt ist Wasserstoffperoxid.

Die Cyclisierung in den Verfahren [A], [B] und [C] wird im allgemeinen in einem Temperaturbereich von 0°C bis +160°C, bevorzugt bei der Siedetemperatur des jeweiligen Lösemittels durchgeführt.

Die Cyclisierung wird im allgemeinen bei Normaldruck durchgeführt. Es ist aber auch möglich, das Verfahren bei Überdruck oder bei Unterdruck durchzuführen (z.B. in einem Bereich von 0.5 bis 5 bar).

Als Lösemittel für das Verfahren [B] eignen sich die oben für den zweiten Schritt der Verfahren [A] und [C] aufgeführten Alkohole, wobei Ethanol bevorzugt ist.

Als Basen für das Verfahren [B] eignen sich Alkalihydride, wie beispielsweise Natrium- oder Kaliumhydrid, oder Alkalialkoholate, wie beispielsweise Natriummethanolat, -ethanolat, -isopropylat oder Kalium-tert.-butylat. Bevorzugt ist Natriumhydrid.

Die Base wird in einer Menge von 2 mol bis 8 mol, bevorzugt von 3 mol bis 6 mol, jeweils bezogen auf 1 mol der Verbindungen der Formel (II), eingesetzt.

Die Verbindungen der Formel (II) sind bekannt oder können beispielsweise hergestellt werden, indem man zunächst Ethoxymethylenmalonsäuredinitril mit Hydrazin-Derivaten der Formel (VII)

R⁴-NH-NH₂ (VII),

in welcher R⁴ die oben angegebenen Bedeutungen hat,
in einem inerten Lösemittel zu den Pyrazolnitrilen der Formel (V) kondensiert und diese dann mit einem der oben aufgeführten Oxidationsmittel, vorzugsweise Wasserstoffperoxid, in Anwesenheit von Ammoniak umsetzt [vgl. z.B. A. Miyashita et al., Heterocycles 1990, 31, 1309ff].

Die Verbindungen der Formeln (IIIa), (IIIb) und (VII) sind kommerziell erhältlich, literaturbekannt oder können in Analogie zu literaturbekannten Verfahren hergestellt werden.

Das erfindungsgemäße Verfahren kann durch das folgendes Formelschema beispielhaft erläutert werden:

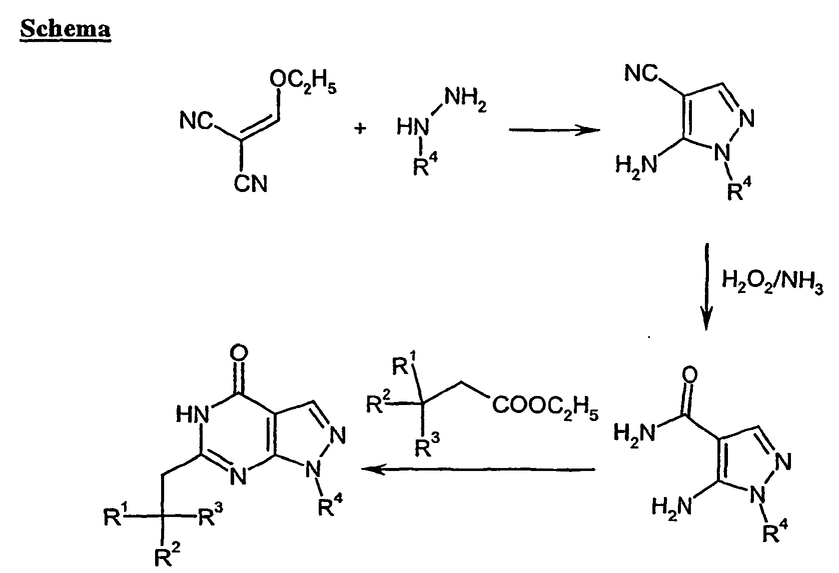

Verbindungen der Formel (IA) und (IB) können gegebenenfalls im Bedeutungsumfang von R¹, R², und R³ nach Standardverfahren weiter modifiziert werden.

Weitere Verfahren zur Herstellung von Pyrazolo[3,4-d]pyrimidin-4-onen sind bekannt und können ebenfalls zur Synthese der erfindungsgemäßen Verbindungen eingesetzt werden (siehe zum Beispiel: P. Schmidt et al., Helvetica Chimica Acta 1962, *189*, 1620ff.).

Die erfindungsgemäßen Verbindungen zeigen ein nicht vorhersehbares, wertvolles pharmakologisches und pharmakokinetisches Wirkspektrum.

Sie eignen sich daher zur Verwendung als Arzneimittel zur Behandlung und/oder Prophylaxe von Krankheiten bei Menschen und Tieren.

Der Begriff "Behandlung" im Rahmen der vorliegenden Erfindung schließt die Prophylaxe ein.

Überraschenderweise wurde gefunden, dass selektive PDE9A-Inhibitoren zur Herstellung von Arzneimitteln zur Verbesserung der Wahrnehmung, Konzentrationsleistung, Lernleistung oder Gedächtnisleistung geeignet sind.

Die erfindungsgemäßen Verbindungen können aufgrund ihrer pharmakologischen Eigenschaften allein oder in Kombination mit anderen Arzneimitteln zur Verbesserung von Wahrnehmung, Konzentrationsleistung, Lern- und/oder Gedächtnisleistung eingesetzt werden.

Weiterer Gegenstand der vorliegenden Offenbarung ist ein Verfahren zur Behandlung und/oder Prophylaxe von Erkrankungen, insbesondere der zuvor genannten Erkrankungen, unter Verwendung einer wirksamen Menge der erfindungsgemäßen Verbindungen.

Ein PDE9A-Inhibitor im Sinne der Erfindung ist eine Verbindung, die humane PDE9A unter den unten angegebenen Bedingungen mit einem IC₅₀-Wert von weniger als 10 µM, bevorzugt weniger als 1 µM hemmt.

Ein selektiver PDE9A-Inhibitor im Sinne der Erfindung ist eine Verbindung, die humane PDE9A unter den unten angegebenen Bedingungen stärker hemmt als die humanen PDE1C, PDE2A, PDE3B, PDE4B, PDE5A, PDE7B, PDE8A, PDE10A und PDE11. Bevorzugt ist ein Verhältnis von IC₅₀ (PDE9A) / IC₅₀ (PDE1C, PDE2A, PDE3B, PDE4B, PDE5A, PDE7B und PDE10A) kleiner als 0.2.

Besonders eignen sich die selektiven PDE9A-Inhibitoren zur Verbesserung der Wahrnehmung, Konzentrationsleistung, Lernleistung, oder Gedächtnisleistung nach Kognitiven Störungen, wie sie insbesondere bei Situationen/Krankheiten/Syndromen auftreten wie "Mild cognitive impairment", Altersassoziierte Lern- und Gedächtnisstörungen, Altersassoziierte Gedächtnisverluste, Vaskuläre Demenz, Schädel-Hirn-Trauma, Schlaganfall, Demenz, die nach Schlaganfällen auftritt ("post stroke dementia"), post-traumatische Demenz, allgemeine Konzentrationsstörungen, Konzentrationsstörungen in Kindern mit Lern- und Gedächtnisproblemen, Alzheimer'sche Krankheit, Demenz mit Lewy-Körperchen, Demenz mit Degeneration der Frontallappen einschließlich des Pick's Syndroms, Parkinson'sche Krankheit, Progressive nuclear palsy, Demenz mit corticobasaler Degeneration, Amyotrope Lateralsklerose (ALS), Huntingtonsche Krankheit, Multiple Sklerose, Thalamische Degeneration, Creutzfeld-Jacob-Demenz, HIV-Demenz, Schizophrenie mit Demenz oder Korsakoff-Psychose.

Die *in vitro*-Wirkung der erfindungsgemäßen Verbindungen kann mit folgenden biologischen Assays gezeigt werden:

### PDE-Inhibition

Rekombinante PDE1C (GenBank/EMBL Accession Number: NM_005020, Loughney et al. *J. Biol. Chem.* 1996 *271,* 796-806), PDE2A (GenBank/EMBL Accession Number: NM_002599, Rosman et al. *Gene* 1997 *191,* 89-95), PDE3B (GenBank/EMBL Accession Number: NM_000922, Miki et al. *Genomics* 1996, *36,* 476-485), PDE4B (GenBank/EMBL Accession Number: NM_002600, Obemolte et al. *Gene.* 1993, *129,* 239-247), PDE5A (GenBank/EMBL Accession Number: NM_001083, Loughney et al. *Gene* 1998, *216*, 139-147), PDE7B (GenBank/EMBL Accession Number: NM_018945, Hetman et al. *Proc. Natl. Acad Sci. U.S.A.* 2000, *97*, 472-476), PDE8A (GenBank/EMBL Accession Number: AF_056490, Fisher et al. *Biochem. Biophys. Res. Commun.* 1998 246, 570-577), PDE9A (Fisher et al., J. Biol. Chem, 1998, 273 (25): 15559-15564), PDE10A (GenBank/EMBL Accession Number: NM_06661, Fujishige et al. *J Biol Chem*. 1999, *274*, 18438-45.), PDE11A (GenBank/EMBL Accession Number: NM_016953, Fawcett et al. *Proc. Natl. Acad. Sci*. 2000, *97*, 3702-3707) wurden mit Hilfe des pFASTBAC Baculovirus Expressionssystems (GibcoBRL) in Sf9-Zellen exprimiert.

Die Testsubstanzen werden zur Bestimmung ihrer *in vitro* Wirkung an PDE 9A in 100% DMSO aufgelöst und seriell verdünnt. Typischerweise werden Verdünnungsreihen von 200 µM bis 1.6 µM hergestellt (resultierende Endkonzentrationen im Test: 4 µM bis 0.032 µM). Jeweils 2 µL der verdünnten Substanzlösungen werden in die Vertiefungen von Mikrotiterplatten (Isoplate; Wallac Inc., Atlanta, GA) vorgelegt. Anschließend werden 50 µL einer Verdünnung des oben beschriebenen PDE9A-Präparates hinzugefügt. Die Verdünnung des PDE9A-Präparates wird so gewählt, dass während der späteren Inkubation weniger als 70% des Substrates umgesetzt wird (typische Verdünnung: 1: 10000; Verdünnungspuffer: 50 mM Tris/HCl pH 7.5, 8.3 mM MgCl₂, 1.7 mM EDTA, 0.2% BSA). Das Substrat, [8-³H] guanosine 3',5'-cyclic phosphate (1 µCi/µL; Amersham Pharmacia Biotech., Piscataway, NJ) wird 1:2000 mit Assaypuffer (50 mM Tris/HCl pH 7.5, 8.3 mM MgCl₂, 1.7 mM EDTA) auf eine Konzentration von 0.0005 µCi/µL verdünnt. Durch Zugabe von 50 µL (0.025 µCi) des verdünnten Substrates wird die Enzymreaktion schließlich gestartet. Die Testansätze werden für 60 min bei Raumtemperatur inkubiert und die Reaktion durch Zugabe von 25 µl eines in Assaypuffer gelösten PDE9A-Inhibitors (z.B. der Inhibitor aus Herstellbeispiel 1, 10 µM Endkonzentration) gestoppt. Direkt im Anschluss werden 25 µL einer Suspension mit 18 mg/mL Yttrium Scintillation Proximity Beads (Amersham Pharmacia Biotech., Piscataway, NJ) hinzugefügt. Die Mikrotiterplatten werden mit einer Folie versiegelt und für 60 min bei Raumtemperatur stehengelassen. Anschließend werden die Platten für 30 s pro Vertiefung in einem Microbeta Szintillationzähler (Wallac Inc., Atlanta, GA) vermessen. IC₅₀₋Werte werden anhand der graphischen Auftragung der Substanzkonzentration gegen die prozentuale Inhibition bestimmt.

Die *in vitro* Wirkung von Testsubstanzen an rekombinanter PDE3B, PDE4B, PDE7B, PDE8A, PDE10A und PDE11A wird nach dem oben für PDE 9A beschriebenen Testprotokoll mit folgenden Anpassungen bestimmt: Als Substrat wird [5',8-³H] adenosine 3',5'-cyclic phosphate (1 µCi/µL; Amersham Pharmacia Biotech., Piscataway, NJ) verwendet. Die Zugabe einer Inhibitorlösung zum Stoppen der Reaktion ist nicht notwendig. Stattdessen wird in Anschluß an die Inkubation von Substrat und PDE direkt mit der Zugabe der Yttrium Scintillation Proximity Beads wie oben beschrieben fortgefahren und dadurch die Reaktion gestoppt. Für die Bestimmung einer entsprechenden Wirkung an rekombinanter PDE1C, PDE2A und PDE5A wird das Protokoll zusätzlich wie folgt angepaßt: Bei PDE1C werden zusätzlich Calmodulin 10⁻⁷ M und CaCl₂ 3 mM zum Reaktionsansatz gegeben. PDE2A wird im Test durch Zugabe von cGMP 1 µM stimuliert und mit einer BSA-Konzentration von 0.01 % getestet. Für PDE1C und PDE2A wird als Substrat [5',8-³H] adenosine 3',5'-cyclic phosphate (1 µCi/µL; Amersham Pharmacia Biotech., Piscataway, NJ), für PDE5A [8-³H] guanosine 3',5'-cyclic phosphate (1 µCi/µL; Amersham Pharmacia Biotech., Piscataway, NJ) eingesetzt.

Die PDE9A-inhibierende Wirkung der erfindungsgemäßen Verbindungen kann anhand der folgenden Beispiele in den Tabellen 1 und 2 gezeigt werden:

**Tabelle 1: Inhibition von PDE-Isoenzymen durch Beispiel 3**

| **Isoenzym** | **Species** | **IC**_{**50**} **[nM]** |
|---|---|---|
| PDE1C | human | 720 |
| PDE2A | human | > 4000 |
| PDE3B | human | > 4000 |
| PDE4B | human | > 4000 |
| PDE5A | human | > 4000 |
| PDE7B | human | > 4000 |
| PDE8A | human | > 4000 |
| PDE9A | human | 110 |
| PDE10A | human | > 4000 |

**Tabelle 2: PDE9A-inhibierende Wirkung der erfindungsgemäßen Verbindungen**

| **Beispiel** | **IC**_{**50**} **[nM]** |
|---|---|
| 1 | 5 |
| 3 | 110 |
| 4 | 30 |
| 6 | 6 |
| 12 | 65 |
| 17 | 86 |
| 19 | 390 |

### Erhöhung der intrazellulären neuronalen cGMP-Konzentration in Zellkulturen

PDE9A-Inhibitoren erhöhen die intrazelluläre neuronale cGMP in kultivierten primären kortikalen Neuronen.

Rattenembryonen (Embryonaltag E17 - E19) wurden dekapitiert, die Köpfe in mit Präparationsmedium (DMEM, Penicillin/Streptomycin; beides von Gibco) gefüllte Präparationsschalen überführt. Die Kopfhaut und Schädeldecke wurde entfernt, und die freipräparierten Gehirne wurden in eine weitere Petrischale mit Präparationsmedium überführt. Mithilfe eines Binokulars und zweier Pinzetten wurde das Großhirn (Kortex) isoliert und mit Eis auf 4°C gekühlt. Diese Präparation und die Vereinzelung der kortikalen Neuronen wurden dann nach einem Standardprotokoll mit dem Papain-Kit (Worthington Biochemical Corporation, Lakewood, New Jersey 08701, USA) durchgeführt (Huettner et al. *J. Neurosci.* 1986, *6*, 3044-3060). Die mechanisch vereinzelten kortikalen Neurone wurden zu 150.000 Zellen/Loch in 200 µl Neurobasalmedium/Loch (Neurobasal; B27 Supplement; 2 mM L-Glutamin; in Anwesenheit von Penicillin/Streptomycin; alle Agenzien von Gibco) 7 Tage in 96 Lochplatten (mit Poly-D-Lysin 100 µg/ml für 30 min vorbehandelt) unter Standard-Bedingungen kultiviert (37°C, 5 % CO₂). Nach 7 Tagen wurde das Medium abgenommen und die Zellen mit HBSS-Puffer (Hank's balanced salt solution, Gibco/BRL) gewaschen. Anschließend werden 100 µl erfindungsgemäße Verbindung in HBSS-Puffer gelöst (zuvor in 100% DMSO gelöst: 10 mM) auf die Zellen gegeben. Anschließend werden nochmals 100 µl HBSS-Puffer zugegeben, sodass die Endkonzentration der erfindungsgemäßen Verbindungen beispielsweise in einem Bereich von 20 nM bis 10 µM liegt, und bei 37°C für 20 min inkubiert. Der Testpuffer wird danach komplett abgenommen. Anschließend werden die Zellen in 200 µl Lysispuffer (cGMP Kit code RPN 226; von Amersham Pharmacia Biotech.) lysiert und die cGMP-Konzentration nach den Angaben des Herstellers gemessen. Alle Messungen werden in Triplikaten durchgeführt. Die statistische Auswertung erfolgt mit Prism Software Version 2.0 (GraphPad Software Inc., San Diego, CA USA).

Inkubation der primären Neuronen mit den erfindungsgemäßen Verbindungen führte zu einer Steigerung des cGMP-Gehaltes.

### Langzeitpotenzierung

Langzeitpotenzierung wird als ein zelluläres Korrelat für Lern- und Gedächtnisvorgänge angesehen. Zur Bestimmung, ob PDE9-Inhibition einen Einfluss auf Langzeitpotenzierung hat, kann folgende Methode angewandt werden:

Rattenhippokampi werden in einen Winkel von etwa 70 Grad im Verhältnis zur Schnittklinge platziert (Chopper). In Abständen von 400 µm wird der Hippokampus zerschnitten. Die Schnitte werden mit Hilfe eines sehr weichen, stark benetzten Pinsels (Marderhaar) von der Klinge genommen und in ein Glasgefäß mit carbogenisierter gekühlter Nährlösung (124 mM NaCl, 4.9 mM KCl, 1.3 mM MgSO₄ x 7 H₂O, 2.5 mM CaCl₂ wasserfrei, 1.2 mM KH₂PO₄, 25.6 mM NaHCO₃, 10 mM Glucose, pH 7.4) überführt. Während der Messung befinden sich die Schnitte in einer temperierten Kammer unter einem Flüssigkeitsspiegel von 1-3 mm Höhe. Die Durchflussrate beträgt 2.5 ml/min. Die Vorbegasung erfolgt unter geringen Überdruck (etwa 1 atm) sowie über eine Mikrokanüle in der Vorkammer. Die Schnittkammer ist mit der Vorkammer so verbunden, daß eine Minizirkulation aufrechterhalten werden kann. Als Antrieb der Minizirkulation wird das durch die Mikrokanüle ausströmende Carbogen eingesetzt. Die frisch präparierten Hippokampusschnitte werden mindestens 1 Stunde bei 33°C in der Schnittkammer adaptiert.

Die Reizstärke wird so gewählt, dass die fokalen exzitatiorischen postsynaptischen Potentiale (fEPSP) 30 % des maximalen exzitatorischen postsynaptischen Potentials (EPSP) betragen. Mit Hilfe einer monopolaren Stimulationselektrode, die aus lackiertem Edelstahl besteht, und eines stromkonstanten, biphasischen Reizgenerators (AM-Systems 2100) werden lokal die Schaffer-Kollateralen erregt (Spannung: 1-5 V, Impulsbreite einer Polarität 0.1 ms, Gesamtimpuls 0.2 ms). Mit Hilfe von Glaselektroden (Borosilikatglas mit Filament, 1-5 MOhm, Durchmesser: 1.5 mm, Spitzendurchmesser: 3-20 µm), die mit normaler Nährlösung gefüllt sind, werden aus dem Stratum radiatum die exzitatorischen postsynaptischen Potentiale (fEPSP) registriert. Die Messung der Feldpotentiale geschieht gegenüber einer chlorierten Referenzelektrode aus Silber, die sich am Rande der Schnittkammer befindet, mit Hilfe eines Gleichspannungsverstärkers. Das Filtern der Feldpotentiale erfolgt über einen Low-Pass Filter (5 kHz). Für die statistische Analyse der Experimente wird der Anstieg (slope) der fEPSPs (fEPSP-Anstieg) ermittelt. Die Aufnahme, Analyse und Steuerung des Experimentes erfolgt mit Hilfe eines Softwareprogrammes (PWIN), welches in der Abteilung Neurophysiologie entwickelt worden ist. Die Mittelwertbildung der fEPSP-Anstiegswerte zu den jeweiligen Zeitpunkten und die Konstruktion der Diagramme erfolgt mit Hilfe der Software EXCEL, wobei ein entsprechendes Makro die Aufnahme der Daten automatisiert.

Superfusion der Hippokampusschnitte mit einer 10 µM Lösung der erfindungsgemäßen Verbindungen führt zu einer signifikanten Steigerung der LTP.

### Sozialer Wiedererkennungstest:

Der Soziale Wiedererkennungstest ist ein Lern- und Gedächtnistest. Er misst die Fähigkeit von Ratten, zwischen bekannten und unbekannten Artgenossen zu unterscheiden. Deshalb eignet sich dieser Test zur Prüfung der lern- oder gedächtnisverbessernden Wirkung der erfindungsgemäßen Verbindungen.

Adulte Ratten, die in Gruppen gehalten werden, werden 30 min vor Testbeginn einzeln in Testkäfige gesetzt. Vier min vor Testbeginn wird das Testtier in eine Beobachtungsbox gebracht. Nach dieser Adaptationszeit wird ein juveniles Tier zu dem Testtier gesetzt und 2 min lang die absolute Zeit gemessen, die das adulte Tier das Junge inspiziert (Trial 1). Gemessen werden alle deutlich auf das Jungtier gerichteten Verhaltensweisen, d.h. ano-genitale Inspektion, Verfolgen sowie Fellpflege, bei denen das Alttier einen Abstand von höchstens 1 cm zu dem Jungtier hatte. Danach wird das Juvenile herausgenommen, das Adulte mit einer erfindungsgemäßen Verbindung oder Vehikel behandelt und anschließend in seinen Heimkäfig zurückgesetzt. Nach einer Retentionszeit von 24 Stunden wird der Test wiederholt (Trial 2). Eine verringerte Soziale Interaktionszeit im Vergleich zu Trial 1 zeigt an, dass die adulte Ratte sich an das Jungtier erinnert.

Die adulten Tiere werden direkt im Anschluss an Trial 1 entweder mit Vehikel (10 % Ethanol, 20 % Solutol, 70 % physiologische Kochsalzlösung) oder 0.1 mg/kg, 0.3 mg/kg, 1.0 mg/kg bzw. 3.0 mg/kg erfindungsgemäßer Verbindung, gelöst in 10 % Ethanol, 20 % Solutol, 70 % physiologische Kochsalzlösung intraperitoneal injiziert. Vehikel behandelte Ratten zeigen keine Reduktion der sozialen Interaktionszeit in Trial 2 verglichen mit Trial 1. Sie haben folglich vergessen, dass sie schon einmal Kontakt mit dem Jungtier hatten. Überraschenderweise ist die soziale Interaktionszeit im zweiten Durchgang nach Behandlung mit den erfindungsgemäßen Verbindungen signifikant gegenüber den Vehikel behandelten reduziert. Dies bedeutet, dass die substanzbehandelten Ratten sich an das juvenile Tier erinnert haben und somit die erfindungsgemäßen Verbindungen eine verbessernde Wirkung auf Lernen und Gedächtnis aufweist.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im allgemeinen hat es sich als vorteilhaft erwiesen, bei parenteraler Applikation pro Tag Mengen von etwa 0.001 bis 10 mg/kg Körpergewicht zur Erzielung wirksamer Ergebnisse zu verabreichen. Bei oraler Applikation beträgt die Menge pro Tag etwa 0.005 bis 3 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Verwendete Abkürzungen:

- BSA: Rinderserum-Albumin
- DCI: direkte chemische Ionisation (bei MS)
- DMSO: Dimethylsulfoxid
- d.Th.: der Theorie (bei Ausbeute)
- EDTA: Ethylendiamintetraessigsäure
- equiv.: Äquivalent(e)
- ESI: Elektrospray-Ionisation (bei MS)
- HATU: *O*-(7-Azabenzotriazol-1-yl)-*N*,*N*,*N*',*N*'-tetramethyluronium-Hexafluorophosphat
- Lit.: Literatur(stelle)
- MS: Massenspektroskopie
- NMR: Kernresonanzspektroskopie
- Smp.: Schmelzpunkt
- Tris: Tris-(hydroxymethyl)-aminomethan

### Ausgangsverbindungen:

### Beispiel 1A

### 5-Amino-1-cyclohexyl-1H-pyrazol-4-carbonitril

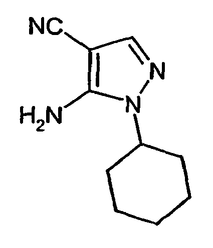

Eine Lösung von Cyclohexylhydrazin-Hydrochlorid (3 g, 19.9 mmol) in 36 ml Ethanol wird bei Raumtemperatur zunächst mit Ethoxymethylenmalonsäuredinitril (2.43 g, 19.9 mmol) und anschließend mit 8 ml Triethylamin versetzt. Das Gemisch wird 20 min refluxiert und dann abgekühlt. Das Lösungsmittel wird am Rotationsverdampfer abgezogen und der Rückstand in DCM aufgenommen, mit wässriger Natriumhydrogencarbonat-Lösung gewaschen, über Natriumsulfat getrocknet, filtriert und im Vakuum eingeengt. Das Rohprodukt wird an Kieselgel chromatographiert (Laufmittel: Dichlormethan/Methanol 0-10 %).
Ausbeute: 1.95 g (51 % d.Th.)
MS (DCI): m/z =191 (M+H)⁺
¹H-NMR (200 MHz, DMSO-d₆): δ = 7.5 (s, 1H), 6.5 (s, 2H), 4.0 (m, 1H), 1.95-1.05 (m, 10H) ppm.

### Beispiel 2A

### 5-Amino-1-cyclopentyl-1H-pyrazol-4-carbonitril

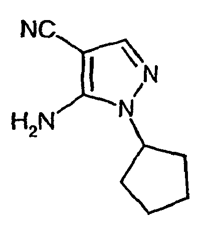

Die Herstellung erfolgt analog der Vorschrift für Beispiel 1A.
MS (ESI): m/z = 177 (M+H)⁺
¹H-NMR (200 MHz, CDCl₃): δ = 7.5 (s, 1H), 4.45 (br. s, 2H), 4.35 (m, 1H), 2.2-1.55 (m, 6H) ppm.

### Beispiel 3A

### 5-Amino-1-(1-ethylpropyl)-1H-pyrazol-4-carbonitril

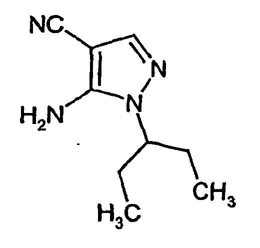

Die Herstellung erfolgt analog der Vorschrift für Beispiel 1A.
MS (ESI): m/z = 179 (M+H)⁺
¹H-NMR (300 MHz, DMSO-d₆): δ = 7.55 (s, 1H), 6.45 (s, 2H), 4.0 (m, 1H), 1.8-1.55 (m, 4H), 0.65 (t, 6H) ppm.

### Beispiel 4A

### 5-Amino-1-cyclohexyl-1H-pyrazol-4-carboxamid

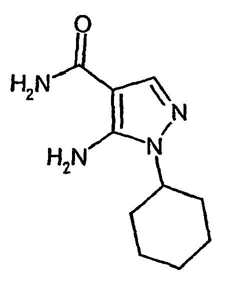

Eine Lösung von 5-Amino-1-cyclohexyl-1H-pyrazol-4-carbonitril (1.86 g, 9.81 mmol) in einem Gemisch aus 73 ml Ethanol und 90 ml konzentrierter wässriger Ammoniaklösung wird bei Raumtemperatur mit 18 ml 30 %-iger Wasserstoffperoxidlösung versetzt und 1 h bei Raumtemperatur gerührt. Anschließend werden am Rotationsverdampfer die nichtwässrigen Lösemittel abgezogen. Aus der verbleibenden Mischung fällt das Produkt als Feststoff aus, der abgesaugt, mit wenig Wasser gewaschen und im Hochvakuum getrocknet wird.
Ausbeute: 1.77 g (86 % d.Th.)
MS (DCI): m/z = 209 (M+H)⁺
¹H-NMR (300 MHz, DMSO-d₆): δ = 7.6 (s, 1H), 7.3-6.4 (breit, 2H), 6.1 (s, 2H), 3.95 (m, 1H), 1.95-1.05 (m, 10H) ppm.

### Beispiel 5A

### 5-Amino-1-cyclopentyl-1H-pyrazol-4-carboxamid

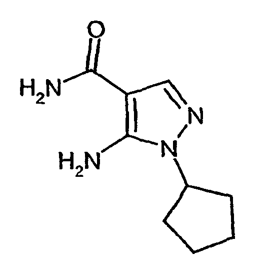

Die Herstellung erfolgt analog der Vorschrift für Beispiel 4A.
MS (ESI): m/z = 195 (M+H)⁺
¹H-NMR (200 MHz, CDCl₃): δ = 7.5 (s, 1H), 5.6-4.8 (breit, 4H), 4.35 (m, 1H), 2.2-1.55 (m, 8H) ppm.

### Beispiel 6A

### 5-Amino-1-(1-ethylpropyl)-1H-pyrazol-4-carboxamid

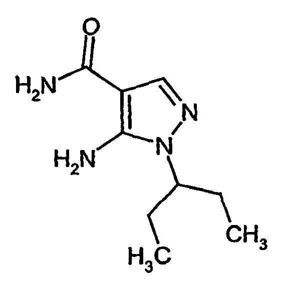

Die Herstellung erfolgt analog der Vorschrift für Beispiel 4A.
MS (ESI): m/z = 197 (M+H)⁺
¹H-NMR (300 MHz, DMSO-d₆): δ = 7.65 (s, 1H), 6.9 (br. s, 2H), 6.1 (s, 2H), 3.9 (m, 1H), 1.85-1.6 (m, 4H), 0.7 (t, 6H) ppm.

### Ausführungsbeispiele:

### Beispiel 1

### 6-(Cyclohexylmethyl)-1-cyclopentyl-1,5-dihydro-4H-pyrazolo[3,4-d]pyrimidin-4-on

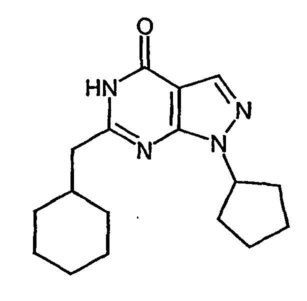

Unter Argon werden 75 mg (0.39 mmol) 5-Amino-1-cyclopentyl-1H-pyrazol-4-carboxamid und 183 mg (1.16 mmol, 3 equiv.) Cyclohexylessigsäuremethylester in 1.5 ml absolutem Ethanol vorgelegt. Bei 0°C werden 54 mg Natriumhydrid (60 %-ige Dispersion in Mineralöl; 1.35 mmol, 3.5 equiv.) im Argon-Gegenstrom langsam zugegeben. Das entstandene Gemisch wird langsam erwärmt und für 18 h unter Rückfluss gerührt. Zur Aufarbeitung werden 20 ml Wasser zugegeben und das Gemisch mehrmals mit Essigsäureethylester extrahiert. Die vereinigten organischen Phasen werden über Natriumsulfat getrocknet und im Vakuum eingeengt. Das Rohprodukt wird mittels präparativer HPLC gereinigt.
Ausbeute: 36 mg (31% d.Th.)
MS (ESI): m/z = 301 (M+H)⁺
Smp.: 147°C
¹H-NMR (300 MHz, DMSO-d₆): δ = 11.95 (s, 1H), 8.0 (s, 1H), 5.1 (m, 1H), 2.5 (d, 2H), 2.15-1.75 (m, 7H), 1.75-1.55 (m, 7H), 1.3-0.9 (m, 5H) ppm.

### Beispiel 2

### 1-Cyclopentyl-6-(3-hydroxypropyl)-1,5-dihydro-4H-pyrazolo[3,4-d]pyrimidin-4-on

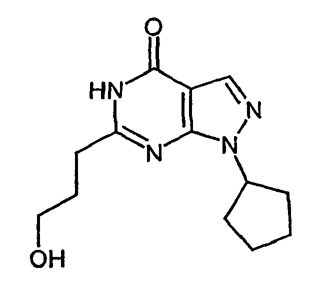

Analog zu Beispiel 1 wird das Produkt ausgehend von 75 mg (0.39 mmol) 5-Amino-1-cyclopentyl-1H-pyrazol-4-carboxamid und 140 mg (1.16 mmol) 4-Hydroxybuttersäuremethylester erhalten.
Ausbeute: 85 mg (84 % d.Th.)
MS (DCI): m/z = 263 (M+H)⁺
Smp.: 138°C
¹H-NMR (200 MHz, DMSO-d₆): δ = 8.0 (s, 1H), 5.1 (m, 1H), 3.5 (t, 2H, *J =* 6.5 Hz), 2.65 (t, 2H, *J* = 7.5 Hz), 2.2-1.55 (m, 10H) ppm.

### Beispiel 3

### 6-(Cyclohexylmethyl)-1-(1-ethylpropyl)-1,5-dihydro-4H-pyrazolo[3,4-d]pyrimidin-4-on

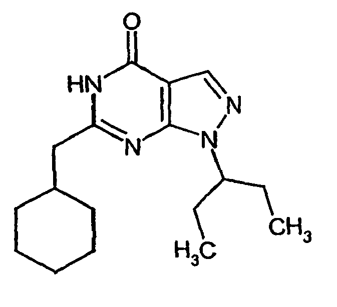

Analog zu Beispiel 1 wird das Produkt ausgehend von 200 mg (1.02 mmol) 5-Amino-1-(1-ethylpropyl)-1H-pyrazol-4-carboxamid und 482 mg (3.06 mmol) Cyclohexylessigsäuremethylester erhalten.
Ausbeute: 146 mg (47 % d.Th.)
MS (ESI): m/z = 303 (M+H)⁺
Smp.: 122°C
¹H-NMR (200 MHz, DMSO-d₆): δ = 12.0 (s, 1H), 8.0 (s, 1H), 4.45 (m, 1H), 2.5 (m, 2H), 2.0-1.5 (m, 10H), 1.4-0.9 (m, 5H), 0.6 (t, 6H, *J* = 7.5 Hz) ppm.

### Beispiel 4

### 1-Cyclopentyl-6-(2-methylbutyl)-1,5-dihydro-4H-pyrazolo[3,4-d]pyrimidin-4-on

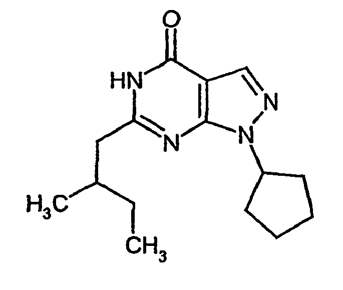

Analog zu Beispiel 1 wird das Produkt ausgehend von 200 mg (1.01 mmol) 5-Amino-1-cyclopentyl-1H-pyrazol-4-carboxamid und 450 mg (3.03 mmol) 3-Methylvaleriansäureethylester erhalten.
Ausbeute: 88 mg (32 % d.Th.)
MS (DCI): m/z = 275 (M+H)⁺
Smp.: 86°C
¹H-NMR (300 MHz, DMSO-d₆): δ = 12.0 (s, 1H), 8.0 (s, 1H), 5.1 (m, 1H), 2.65 (dd, 1H), 2.45 (dd, 1H), 2.15-1.8 (m, 7H), 1.7 (m, 2H), 1.45-1.15 (m, 2H), 0.9 (m, 6H) ppm.

### Beispiel 5

### 1-Cyclopentyl-6-(3-methylbutyl)-1,5-dihydro-4H-pyrazolo[3,4-d]pyrimidin-4-on

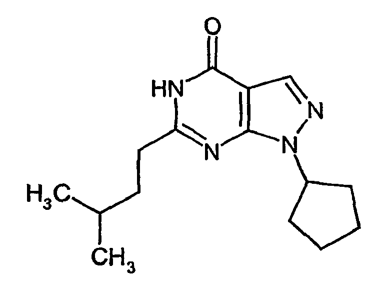

Analog zu Beispiel 1 wird das Produkt ausgehend von 200 mg (1.01 mmol) 5-Amino-1-cyclopentyl-1H-pyrazol-4-carboxamid und 450 mg (3.03 mmol) 4-Methylvaleriansäureethylester erhalten.
Ausbeute: 165 mg (60 % d.Th.)
MS (ESI): m/z = 275 (M+H)⁺
Smp.: 133°C
¹H-NMR (200 MHz, DMSO-d₆): δ = 12.0 (s, 1H), 8.0 (s, 1H), 5.1 (m, 1H), 2.6 (m, 2H), 2.2-1.5 (m, 11H), 0.9 (d, 6H, *J* = 6.5 Hz) ppm.

### Beispiel 6

### 6-(2-Cyclopenten-1-ylmethyl)-1-cyclopentyl-1,5-dihydro-4H-pyrazolo[3,4-d]pyrimidin-4-on

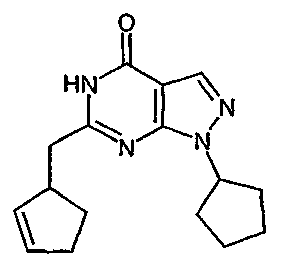

Analog zu Beispiel 1 wird das Produkt ausgehend von 200 mg (1.01 mmol) 5-Amino-1-cyclopentyl-1H-pyrazol-4-carboxamid und 446 mg (1.82 mmol, 95 % rein) 2-Cyclopenten-1-yl-essigsäuremethylester (Lit.: Roenn et al., Tetrahedron Lett. 1995, *36*, 7749) erhalten.
Ausbeute: 86 mg (30 % d.Th.)
MS (ESI): m/z = 285 (M+H)⁺
Smp.: 166°C
¹H-NMR (200 MHz, DMSO-d₆): δ = 12.0 (s, 1H), 8.0 (s, 1H), 5.75 (m, 2H), 5.1 (m, 1H), 3.15 (m, 1H), 2.8-2.5 (m, 2H), 2.45-1.45 (m, 12H) ppm.

### Beispiel 7

### 1-(1-Ethylpropyl)-6-(2-methylbutyl)-1,5-dihydro-4H-pyrazolo[3,4-d]pyrimidin-4-on

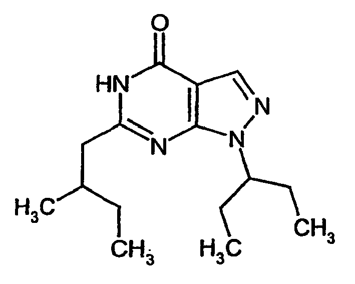

Analog zu Beispiel 1 wird das Produkt ausgehend von 200 mg (1.0 mmol) 5-Amino-1-(1-ethylpropyl)-1H-pyrazol-4-carboxamid und 445 mg (3.0 mmol) 3-Methylvaleriansäureethylester erhalten.
Ausbeute: 99 mg (36 % d.Th.)
MS (ESI): m/z = 277 (M+H)⁺
Smp.: 121°C
¹H-NMR (300 MHz, DMSO-d₆): δ = 12.0 (s, 1H), 8.0 (s, 1H), 4.5 (m, 1H), 2.6 (dd, 1H), 2.45 (dd, 1H), 2.05-1.7 (m, 5H), 1.45-1.15 (m, 2H), 0.9 (m, 6H), 0.65 (t, 6H, *J* = 7.5 Hz) ppm.

### Beispiel 8

### 1-(1-Ethylpropyl)-6-isopentyl-1,5-dihydro-4H-pyrazolo[3,4-d]pyrimidin-4-on

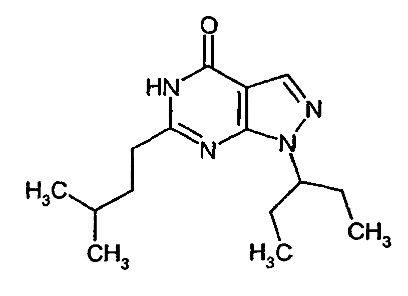

Analog zu Beispiel 1 wird das Produkt ausgehend von 200 mg (1.0 mmol) 5-Amino-1-(1-ethylpropyl)-1H-pyrazol-4-carboxamid und 445 mg (3.0 mmol) 4-Methylvaleriansäureethylester erhalten.
Ausbeute: 127 mg (46 % d.Th.)
MS (ESI): m/z = 277 (M+H)⁺
Smp.: 127°C
¹H-NMR (300 MHz, DMSO-d₆): δ = 12.0 (s, 1H), 8.0 (s, 1H), 4.45 (m, 1H), 2.65 (m, 2H), 2.0-1.8 (m, 4H), 1.7-1.5 (m, 3H), 0.9 (d, 6H, *J* = 7 Hz), 0.6 (t, 6H, *J* = 6 Hz) ppm.

### Beispiel 9

### 1-(1-Ethylpropyl)-6-isobutyl-1,5-dihydro-4H-pyrazolo[3,4-d]pyrimidin-4-on

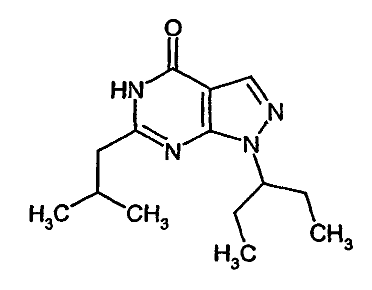

Analog zu Beispiel 1 wird das Produkt ausgehend von 200 mg (1.0 mmol) 5-Amino-1-(1-ethylpropyl)-1H-pyrazol-4-carboxamid und 464 mg (3.5 mmol) 3-Methylbuttersäureethylester erhalten.
Ausbeute: 127 mg (48 % d.Th.)
MS (ESI): m/z = 263 (M+H)⁺
Smp.: 161°C
¹H-NMR (300 MHz, DMSO-d₆): δ = 12.0 (s, 1H), 8.0 (s, 1H), 4.45 (m, 1H), 2.5 (m, 2H), 2.15 (m, 1H), 1.95-1.75 (m, 4H), 0.9 (d, 6H, *J* = 7 Hz), 0.55 (t, 6H, *J* = 7.5 Hz) ppm.

### Beispiel 10

### 1-(1-Ethylpropyl)-6-propyl-1,5-dihydro-4H-pyrazolo[3,4-d]pyrimidin-4-on

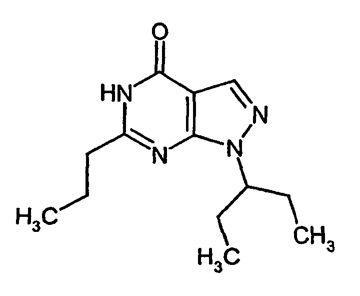

Analog zu Beispiel 1 wird das Produkt ausgehend von 200 mg (1.0 mmol) 5-Amino-1-(1-ethylpropyl)-1H-pyrazol-4-carboxamid und 410 mg (3.5 mmol) Buttersäureethylester erhalten.
Ausbeute: 159 mg (64 % d.Th.)
MS (ESI): m/z = 249 (M+H)⁺
Smp.: 127°C
¹H-NMR (300 MHz, DMSO-d₆): δ = 11.95 (s, 1H), 8.0 (s, 1H), 4.5 (m, 1H), 2.6 (t, 2H, *J* = 7.5 Hz), 2.0-1.65 (m, 6H), 0.9 (t, 3H, *J =* 7.5 Hz), 0.6 (t, 6H, *J* = 7.5 Hz) ppm.

### Beispiel 11

### 1-(1-Ethylpropyl)-6-(tetrahydro-2-furanylmethyl)-1,5-dihydro-4H-pyrazolo[3,4-d]-pyrimidin-4-on

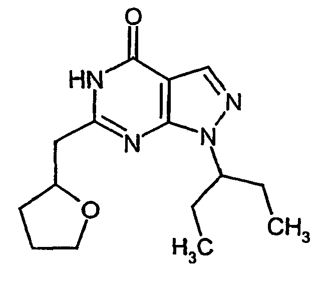

Analog zu Beispiel 1 wird das Produkt ausgehend von 200 mg (1.0 mmol) 5-Amino-1-(1-ethylpropyl)-1H-pyrazol-4-carboxamid und 553 mg (3.5 mmol) Tetrahydrofuran-2-ylessigsäureethylester erhalten.
Ausbeute: 202 mg (68 % d.Th.)
MS (ESI): m/z = 291 (M+H)⁺
Smp.: 136°C
¹H-NMR (200 MHz, DMSO-d₆): δ = 12.0 (s, 1H), 8.0 (s, 1H), 4.45 (m, 1H), 4.25 (m, 1H), 3.75 (m, 1H), 3.6 (m, 1H), 2.8 (m, 2H), 2.1-1.55 (m, 8H), 0.6 (t, 6H, *J* = 7.5 Hz) ppm.

### Beispiel 12

### 6-(2-Cyclopenten-1-ylmethyl)-1-(1-ethylpropyl)-1,5-dihydro-4H-pyrazolo[3,4-d]-pyrimidin-4-on

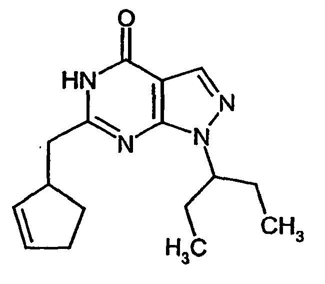

Analog zu Beispiel 1 wird das Produkt ausgehend von 200 mg (1.0 mmol) 5-Amino-1-(1-ethylpropyl)-1H-pyrazol-4-carboxamid und 490 mg (3.5 mmol) 2-Cyclopenten-1-ylessigsäuremethylester (Lit.: Roenn et al., Tetrahedron Lett. 1995, 36, 7749) erhalten.
Ausbeute: 111 mg (39 % d.Th.)
MS (ESI): m/z = 287 (M+H)⁺
Smp.: 128°C
¹H-NMR (200 MHz, DMSO-d₆): δ = 12.0 (s, 1H), 8.0 (s, 1H), 5.8-5.65 (m, 2H), 4.5 (m, 1H), 3.2 (m, 1H), 2.8-2.55 (m, 2H), 2.3 (m, 2H), 2.15-1.8 (m, 5H), 1.55 (m, 1H), 0.65 (t, 6H, *J* = 7.5 Hz) ppm.

### Beispiel 13

### 4-[1-(1-Ethylpropyl)-4-oxo-4,5-dihydro-1H-pyrazolo[3,4-d]pyrimidin-6-yl]buttersäureethylester

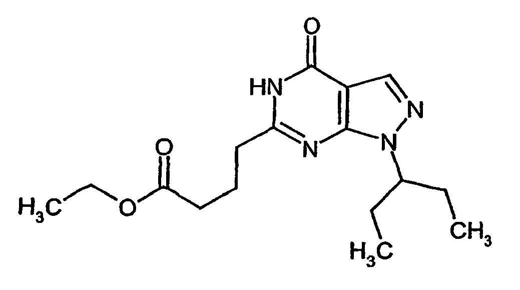

Analog zu Beispiel 1 wird das Produkt ausgehend von 200 mg (1.0 mmol) 5-Amino-1-(1-ethylpropyl)-1H-pyrazol-4-carboxamid und 1.13 g (6.0 mmol) Glutarsäurediethylester erhalten.
Ausbeute: 46 mg (13 % d.Th.)
MS (ESI): m/z = 321 (M+H)⁺
¹H-NMR (200 MHz, DMSO-d₆): δ = 12.0 (s, 1H), 8.0 (s, 1H), 4.5 (m, 1H), 4.2 (q, 2H, *J =* 7 Hz), 2.7 (t, 2H, *J =* 7.5 Hz), 2.4 (t, 2H, *J =* 7 Hz), 2.1-1.75 (m, 6H), 1.2 (t, 3H, *J =* 7 Hz), 0.65 (t, 6H, *J =* 7.5 Hz) ppm.

### Beispiel 14

### 4-[1-(1-Ethylpropyl)-4-oxo-4,5-dihydro-1H-pyrazolo[3,4-d]pyrimidin-6-yl]propionsäureethylester

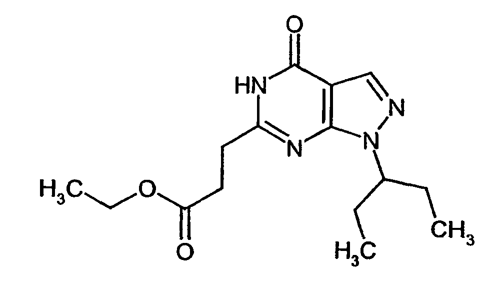

Analog zu Beispiel 1 wird das Produkt ausgehend von 200 mg (1.0 mmol) 5-Amino-1-(1-ethylpropyl)-1H-pyrazol-4-carboxamid und 1.04 g (6 mmol) Bemsteinsäurediethylester erhalten.
Ausbeute: 176 mg (56 % d.Th.)
MS (ESI): m/z = 307 (M+H)⁺
Smp.: 118°C
¹H-NMR (200 MHz, DMSO-d₆): δ = 12.1 (s, 1H), 8.0 (s, 1H), 4.4 (m, 1H), 4.0 (q, 2H, *J =* 7 Hz), 2.9 (m, 2H), 2.8 (m, 2H), 2.0-1.7 (m, 4H), 1.2 (t, 3H, *J =* 7 Hz), 0.6 (t, 6H, *J* = 7.5 Hz) ppm.

### Beispiel 15

### 1-Cyclopentyl-6-[(4-methylcyclohexyl)methyl]-1,5-dihydro-4H-pyrazolo[3,4-d]-pyrimidin-4-on

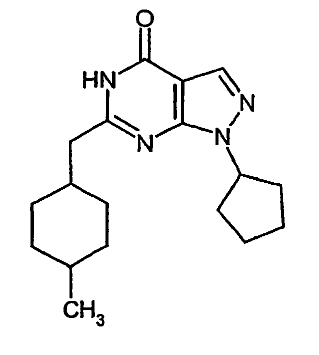

Analog zu Beispiel 1 wird das Produkt ausgehend von 200 mg (1.0 mmol) 5-Amino-1-cyclopentyl-1H-pyrazol-4-carboxamid und 664 mg (3.5 mmol) (4-Methylcyclohexyl)essigsäureethylester (cis/trans-Gemisch) erhalten. Das Produkt liegt als Gemisch der cis- und trans-Isomere vor.
Ausbeute: 131 mg (41 % d.Th.)
MS (ESI): m/z = 315 (M+H)⁺
Smp.: 126°C
¹H-NMR (200 MHz, DMSO-d₆): δ = 12.0 (s, 1H), 8.0 (s, 1H), 5.1 (m, 1H), 2.6 (d, 2H, *J* = 7 Hz), 2.2-0.8 (m, 21H) ppm.

### Beispiel 16

### 1-(1-Ethylpropyl)-6-[(4-methylcyclohexyl)methyl]-1,5-dihydro-4H-pyrazolo[3,4-d]-pyrimidin-4-on

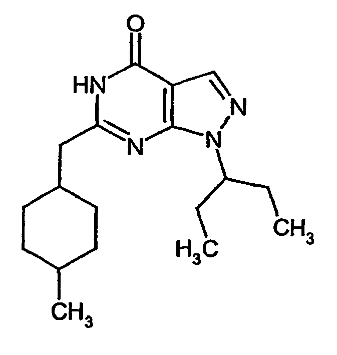

Analog zu Beispiel 1 wird das Produkt ausgehend von 200 mg (1.0 mmol) 5-Amino-1-(1-ethylpropyl)-1H-pyrazol-4-carboxamid und 413 mg (2.2 mmol) (4-Methylcyclohexyl)essigsäureethylester (cis/trans-Gemisch) erhalten. Das Produkt liegt als Gemisch der cis- und trans-Isomere vor.
Ausbeute: 60 mg (19 % d.Th.)
MS (ESI): m/z = 317 (M+H)⁺
¹H-NMR (200 MHz, DMSO-d₆): δ = 12.0 (s, 1H), 8.0 (s, 1H), 4.45 (m, 1H), 2.6 (d, 2H, *J* = 7 Hz), 2.2-0.8 (m, 17H), 0.6 (t, 6H, *J =* 7.5 Hz) ppm.

### Beispiel 17

### 1-Cyclopentyl-6-(tetrahydro-2-furanylmethyl)-1,5-dihydro-4H-pyrazolo[3,4-d]pyrimidin-4-on

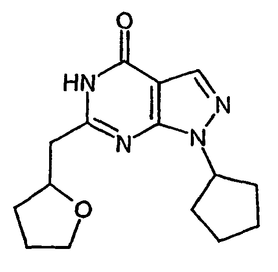

Analog zu Beispiel 1 wird das Produkt ausgehend von 200 mg (1.0 mmol) 5-Amino-1-(1-ethylpropyl)-1H-pyrazol-4-carboxamid und 559 mg (3.5 mmol) Tetrahydrofuran-2-ylessigsäureethylester erhalten.
Ausbeute: 175 mg (60 % d.Th.)
MS (ESI): m/z = 289 (M+H)⁺
Smp.: 179°C
¹H-NMR (200 MHz, DMSO-d₆): δ = 11.95 (s, 1H), 8.0 (s, 1H), 5.1 (m, 1H), 4.3 (m, 1H), 3.8 (m, 1H), 3.6 (m, 1H), 2.8 (m, 2H), 2.15-1.55 (m, 12H) ppm.

### Beispiel 18

### 4-[1-Cyclopentyl-4-oxo-4,5-dihydro-1H-pyrazolo[3,4-d]pyrimidin-6-yl]propionsäureethylester

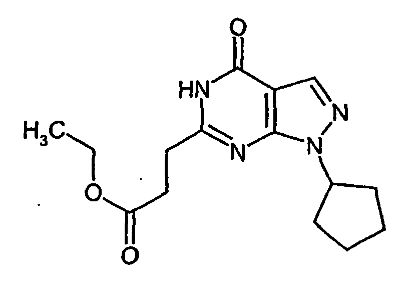

Analog zu Beispiel 1 wird das Produkt ausgehend von 200 mg (1.0 mmol) 5-Amino-1-cyclopentyl-1H-pyrazol-4-carboxamid und 1.05 g (6.05 mmol) Bemsteinsäurediethylester erhalten.
Ausbeute: 150 mg (49 % d.Th.)
MS (DCI): m/z = 305 (M+H)⁺
Smp.: 185°C
¹H-NMR (200 MHz, DMSO-d₆): δ = 12.1 (s, 1H), 8.0 (s, 1H), 5.05 (m, 1H), 4.05 (q, 2H, *J =* 7 Hz), 2.9 (m, 2H), 2.8 (m, 2H), 2.15-1.6 (m, 8H), 1.2 (t, 3H, *J =* 7 Hz) ppm.

### Beispiel 19

### 6-(Cyclohexylmethyl)-1-(1-ethylpropyl)-1,5-dihydro-4H-pyrazolo[3,4-d]pyrimidin-4-thion

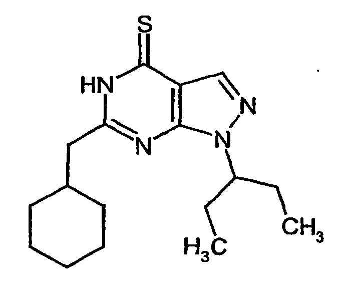

Eine Lösung von 50 mg (0.17 mmol) 6-(Cyclohexylmethyl)-1-(1-ethylpropyl)-1,5-dihydro-4H-pyrazolo[3,4-d]pyrimidin-4-on (Beispiel 3) in 1 ml Pyridin wird bei Raumtemperatur mit 74 mg (0.33 mmol, 2 equiv.) Diphosphorpentasulfid versetzt und anschließend über Nacht unter Rückfluss gerührt. Nach dem Abkühlen wird die Reaktionslösung mit 20 ml eiskalter 2.5 %-iger Natriumhydrogencarbonat-Lösung versetzt und dreimal mit Essigsäureethylester extrahiert. Die vereinigten organischen Phasen werden mit gesättigter Kochsalzlösung gewaschen, über Natriumsulfat getrocknet und im Vakuum eingeengt. Das Rohprodukt wird über präparative HPLC gereinigt.
Ausbeute: 42 mg (80 % d.Th.)
MS (DCI): m/z = 319 (M+H)⁺
¹H-NMR (200 MHz, DMSO-d₆): δ = 13.4 (s, 1H), 8.2 (s, 1H), 4.45 (m, 1H), 2.7 (d, 2H, *J =* 7 Hz), 2.0-1.5 (m, 10H), 1.4-0.85 (m, 5H), 0.6 (t, 6H, *J =* 7.5 Hz) ppm.

### Beispiel 20

### 3-(1-Cyclopentyl-4-oxo-4,5-dihydro-1H-pyrazolo[3,4-d]pyrimidin-6-yl)-N-phenylpropanamid

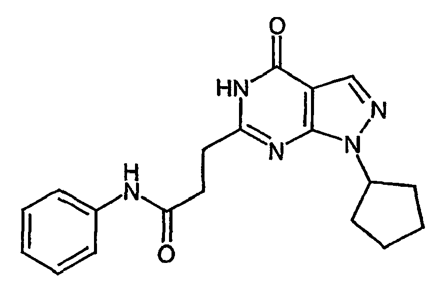

Eine Lösung von 100 mg (0.33 mmol) 4-[1-Cyclopentyl-4-oxo-4,5-dihydro-1H-pyrazolo[3,4-d]pyrimidin-6-yl]propionsäureethylester (Beispiel 18) in einem Gemisch aus 1 ml Ethanol und 0.5 ml 20 %-iger Natronlauge wird 1 h bei 60°C gerührt. Der organische Lösemittelanteil wird am Rotationsverdampfer abgezogen und die Lösung mit 1 N Salzsäure auf pH 3 gestellt. Die Lösung wird danach bis zur Trockne eingedampft, der Rückstand mit 5 ml Methanol verrührt und die Lösung filtriert. Nach Abziehen des Methanols erhält man die entsprechende Carbonsäure als Rohprodukt (90 mg, quantitativ).
87 mg (0.31 mmol) der so erhaltenen Carbonsäure werden in 6 ml Dichlormethan vorgelegt und zunächst mit 119 mg (0.31 mmol, 1 equiv.) HATU und anschließend mit 29 mg (0.31 mmol, 1 equiv.) Anilin und 81 mg (0.63 mmol, 2 equiv.) N-Ethyldiisopropylamin versetzt und über Nacht gerührt. Zur Aufarbeitung wird die Reaktionslösung zweimal mit gesättigter Natriumhydrogencarbonat-Lösung gewaschen, die organische Phase über Natriumsulfat getrocknet und im Vakuum eingeengt. Das Rohprodukt wird mittels präparativer HPLC gereinigt.
Ausbeute: 25 mg (22 % d.Th.)
MS (ESI): m/z = 352 (M+H)⁺
¹H-NMR (200 MHz, DMSO-d₆): δ = 12.05 (s, 1H), 10.1 (s, 1H), 8.0 (s, 1H), 7.6 (d, 2H), 7.3 (t, 2H), 7.0 (t, 1H), 5.0 (m, 1H), 3.0 (m, 2H), 2.8 (m, 2H), 2.05-1.4 (m, 8H) ppm.

### Beispiel 21

### 6-(Cyclopentylmethyl)-1-(1-ethylpropyl)-1,5-dihydro-4H-pyrazolo[3,4-d]pyrimidin-4-on

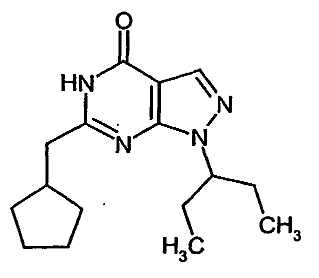

Analog zu Beispiel 1 wird das Produkt ausgehend von 150 mg (0.75 mmol) 5-Amino-1-(1-ethylpropyl)-1H-pyrazol-4-carboxamid und 351 mg (2.25 mmol) 2-Cyclopentylessigsäureethylester erhalten.
Ausbeute: 91 mg (42 % d.Th.)
MS (ESI): m/z = 289 (M+H)⁺
Smp.: 156°C
¹H-NMR (200 MHz, DMSO-d₆): δ = 12.0 (s, 1H), 8.0 (s, 1H), 4.45 (m, 1H), 2.7 (d, 2H, *J* = 7.5 Hz), 2.3 (m, 1H), 2.0-1.45 (m, 10H), 1.35-1.1 (m, 2H), 0.6 (t, 6H, *J* = 7.5 Hz) ppm.

### Beispiel 22

### 1-(1-Ethylpropyl)-6-(3,3,3-trifluor-2-methylpropyl)-1,5-dihydro-4H-pyrazolo[3,4-d]-pyrimidin-4-on

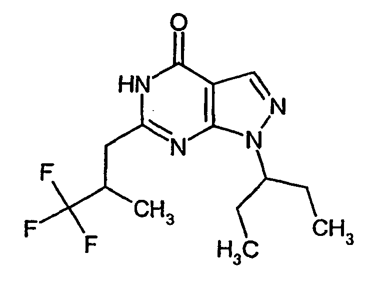

Analog zu Beispiel 1 wird das Produkt ausgehend von 100 mg (0.51 mmol) 5-Amino-1-(1-ethylpropyl)-1H-pyrazol-4-carboxamid und 469 mg (2.55 mmol) 3-Methyl-4,4,4-trifluorbuttersäureethylester erhalten.
Ausbeute: 98 mg (61 % d.Th.)
MS (ESI): m/z = 317 (M+H)⁺
Smp.: 156°C
¹H-NMR (200 MHz, DMSO-d₆): δ = 12.2 (s, 1H), 8.05 (s, 1H), 4.45 (m, 1H), 3.2-2.9 (m, 2H), 2.7 (m, 1H), 2.0-1.7 (m, 4H), 1.1 (d, 3H), 0.6 (t, 6H) ppm.

### Beispiel 23

### 1-(1-Cyclopentyl)-6-(3,3,3-trifluor-2-methylpropyl)-1,5-dihydro-4H-pyrazolo[3,4-d]-pyrimidin-4-on

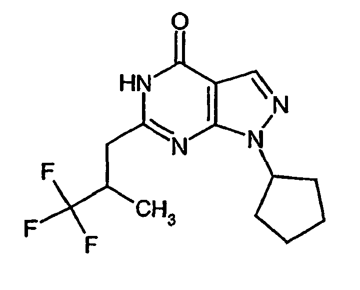

Analog zu Beispiel 1 wird das Produkt ausgehend von 100 mg (0.51 mmol) 5-Amino-1-(1-cyclopentyl)-1H-pyrazol-4-carboxamid und 474 mg (2.57 mmol) 3-Methyl-4,4,4-trifluorbuttersäureethylester erhalten.
Ausbeute: 119 mg (73 % d.Th.)
MS (ESI): m/z = 315 (M+H)⁺
Smp.: 166°C
¹H-NMR (300 MHz, DMSO-d₆): δ = 12.1 (s, 1H), 8.0 (s, 1H), 5. 1 (m, 1H), 3.2-2.95 (m, 2H), 2.7 (m, 1H), 2.2-1.6 (m, 8H), 1.1 (d, 3H) ppm.

## Patentansprüche

1. Verbindungen der Formel 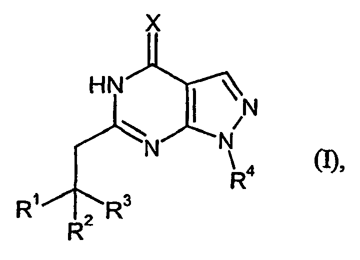
in welcher
R¹ C₁-C₆-Alkyl, Trifluormethyl, Hydroxy, C₁-C₆-Alkoxy, -C(=O)OR⁵ oder -C(=O)NR⁶R⁷, wobei C₁-C₆-Alkyl gegebenenfalls mit 1 bis 3 Resten unabhängig voneinander ausgewählt aus der Gruppe Hydroxy, C₁-C₆-Alkoxy, Halogen, Trifluormethyl, Trifluormethoxy, -C(=O)OR⁵ oder -C(=O)NR⁶R⁷ substituiert ist, und
R⁵ für C₁-C₆-Alkyl,
R⁶ und R⁷ unabhängig voneinander für Wasserstoff, C₆-C₁₀-Aryl, C₁-C₆-Alkyl stehen, oder
zusammen mit dem Stickstoffatom, an das sie gebunden sind, ein 4- bis 10-gliedriges Heterocyclyl bilden,
R² Wasserstoff, C₁-C₆-Alkyl, Trifluormethyl, C₁-C₆-Alkoxy,
oder
R¹ und R² zusammen mit dem Kohlenstoffatom, an das sie gebunden sind, C₃-C₈-Cycloalkyl, C₃-C₈-Cycloalkenyl oder 4- bis 10-gliedriges Heterocyclyl bilden, die gegebenenfalls mit bis zu 2 Substituenten aus der Gruppe C₁-C₆-Alkyl, C₁-C₆-Alkoxy, Hydroxy, Oxo, -C(=O)OR⁸ substituiert sind, und
R⁸ für C₁-C₆-Alkyl oder Benzyl steht,
R³ Wasserstoff oder C₁-C₆-Alkyl,
R⁴ Pentan-3-yl, C₃-C₆-Cycloalkyl,
X Sauerstoff oder Schwefel,
bedeuten, sowie deren Salze, Solvate und/oder Solvate der Salze.

2. Verbindungen nach Anspruch 1, wobei
R¹ C₁-C₆-Alkyl, Hydroxy, C₁-C₆-Alkoxy, -C(=O)OR⁵ oder -C(=O)NR⁶R⁷, wobei C₁-C₆-Alkyl gegebenenfalls mit Hydroxy, C₁-C₆-Alkoxy, -C(=O)OR⁵ oder -C(=O)NR⁶R⁷ substituiert ist, und
R⁵ für C₁-C₆-Alkyl,
R⁶ und R⁷ unabhängig voneinander für Wasserstoff, C₆-C₁₀-Aryl, C₁-C₆-Alkyl stehen, oder
zusammen mit dem Stickstoffatom, an das sie gebunden sind, ein 4- bis 10-gliedriges Heterocyclyl bilden,
R² Wasserstoff, C₁-C₆-Alkyl, C₁-C₆-Alkoxy,
oder
R¹ und R² zusammen mit dem Kohlenstoffatom, an das sie gebunden sind, C₃-C₈-Cycloalkyl, C₃-C₈-Cycloalkenyl oder 4- bis 10-gliedriges Heterocyclyl bilden, die gegebenenfalls mit bis zu 2 Substituenten aus der Gruppe C₁-C₆-Alkyl, C₁-C₆-Alkoxy, Hydroxy, Oxo, -C(=O)OR⁸ substituiert sind, und
R⁸ für C₁-C₆-Alkyl oder Benzyl steht,
R³ Wasserstoff oder C₁-C₆-Alkyl,
R⁴ Pentan-3-yl, C₄-C₆-Cycloalkyl,
X Sauerstoff oder Schwefel,
bedeuten,
sowie deren Salze, Solvate und/oder Solvate der Salze.

3. Verbindungen nach Ansprüchen 1 und 2, wobei
R¹ C₁-C₄-Alkyl, Hydroxy, C₁-C₄-Alkoxy, -C(=O)OR⁵ oder -C(=O)NR⁶R⁷, wobei C₁-C₄-Alkyl gegebenenfalls mit Hydroxy, Trifluormethyl, C₁-C₄-Alkoxy, -C(=O)OR⁵ oder -C(=O)NR⁶R⁷ substituiert ist, und
R⁵ für C₁-C₄-Alkyl,
R⁶ und R⁷ unabhängig voneinander für Wasserstoff, Phenyl, C₁-C₄-Alkyl stehen, oder
zusammen mit dem Stickstoffatom, an das sie gebunden sind, ein 5- bis 6-gliedriges Heterocyclyl bilden,
R² Wasserstoff, C₁-C₄-Alkyl, Trifluormethyl, C₁-C₄-Alkoxy,
oder
R¹ und R² zusammen mit dem Kohlenstoffatom, an das sie gebunden sind, C₅-C₆-Cycloalkyl, C₅-C₆-Cycloalkenyl oder 5- bis 6-gliedriges Heterocyclyl bilden, die gegebenenfalls mit bis zu 2 Substituenten aus der Gruppe C₁-C₄-Alkyl, C₁-C₄-Alkoxy, Hydroxy, Oxo, -C(=O)OR⁸ substituiert sind, und
R⁸ für C₁-C₄-Alkyl oder Benzyl steht,
R³ Wasserstoff,
R⁴ Pentan-3-yl, C₅-C₆-Cycloalkyl,
X Sauerstoff oder Schwefel,
bedeuten,
sowie deren Salze, Solvate und/oder Solvate der Salze.

4. Verbindungen nach Ansprüchen 1 bis 3, wobei
R¹ Methyl, Ethyl, Isopropyl, Trifluormethyl, Methoxycarbonyl, Ethoxycarbonyl oder -C(=O)NR⁶R⁷, wobei Methyl gegebenenfalls mit Methoxycarbonyl oder Ethoxycarbonyl substituiert ist, und
R⁶ für Phenyl steht und
R⁷ für Wasserstoff steht,
R² Wasserstoff, Methyl, Trifluormethyl, oder
R¹ und R² zusammen mit dem Kohlenstoffatom, an das sie gebunden sind, Cyclopentyl, Cyclohexyl, Cyclopentenyl oder Tetrahydrofuryl bilden, wobei Cyclohexyl gegebenenfalls mit Methyl substituiert ist, und
R³ Wasserstoff,
R⁴ Pentan-3-yl, C₅-C₆-Cycloalkyl,
X Sauerstoff oder Schwefel,
bedeuten,
sowie deren Salze, Solvate und/oder Solvate der Salze.

5. Verbindungen nach Ansprüchen 1 bis 4, wobei
R¹ Methyl, Ethyl, Isopropyl, Methoxycarbonyl, Ethoxycarbonyl oder -C(=O)NR⁶R⁷, wobei Methyl gegebenenfalls mit Methoxycarbonyl oder Ethoxycarbonyl substituiert ist, und
R⁶ für Phenyl und
R⁷ für Wasserstoff stehen,
R² Wasserstoff, Methyl, oder
R¹ und R² zusammen mit dem Kohlenstoffatom, an das sie gebunden sind, Cyclopentyl, Cyclohexyl, Cyclopentenyl oder Tetrahydrofuryl bilden, wobei Cyclohexyl gegebenenfalls mit Methyl substituiert ist, und
R³ Wasserstoff,
R⁴ Pentan-3-yl, C₅-C₆-Cycloalkyl,
X Sauerstoff,
bedeuten,
sowie deren Salze, Solvate und/oder Solvate der Salze.

6. Verfahren zur Herstellung von Verbindungen nach Ansprüchen 1 bis 5, **dadurch gekennzeichnet, dass** man
[A] Verbindungen der Formel 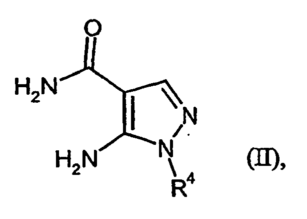
in welcher R⁴ die oben angegebenen Bedeutungen hat,
durch Umsetzung mit einer Verbindung der Formel 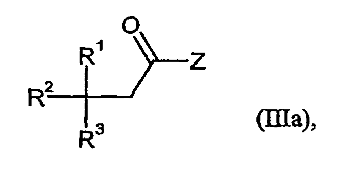
in welcher R¹, R² und R³ die oben angegebenen Bedeutungen haben,
und
Z für Chlor oder Brom steht,
in einem inerten Lösemittel und in Anwesenheit einer Base zunächst in Verbindungen der Formel 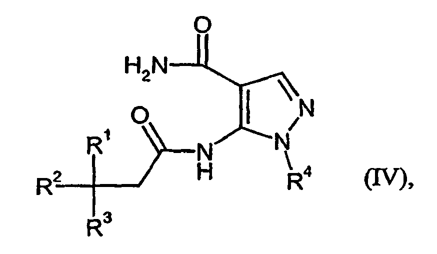
in welcher R¹, R², R³ und R⁴ die oben angegebenen Bedeutungen haben,
überführt,
dann in einem inerten Lösemittel in Gegenwart einer Base zu Verbindungen der Formel 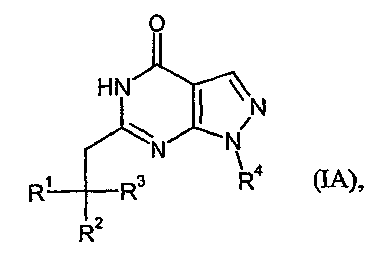
in welcher R¹, R², R³ und R⁴ die oben angegebenen Bedeutungen haben,
cyclisiert,
oder
[B] Verbindungen der Formel (II) unter direkter Cyclisierung zu (IA) mit einer Verbindung der Formel 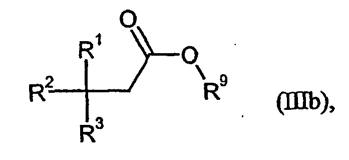
in welcher R¹, R² und R³ die oben angegebenen Bedeutungen haben
und
R⁹ für Methyl oder Ethyl steht,
in einem inerten Lösemittel und in Anwesenheit einer Base umsetzt,
oder
[C] Verbindungen der Formel 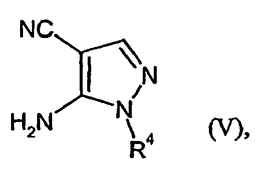
in welcher R⁴ die oben angegebenen Bedeutungen hat,
zunächst durch Umsetzung mit einer Verbindung der Formel (IIIa) in einem inerten Lösemittel und in Anwesenheit einer Base in Verbindungen der Formel 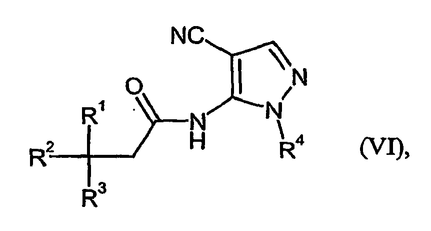
in welcher R¹, R², R³ und R⁴ die oben angegebenen Bedeutungen haben,
überführt,
und diese in einem zweiten Schritt in einem inerten Lösemittel und in Anwesenheit einer Base und eines Oxidationsmittels zu (IA) cyclisiert,
und die Verbindungen der Formel (IA) gegebenenfalls dann durch Umsetzung mit einem Schwefelungsmittel wie beispielsweise Diphosphorpentasulfid in die Thiono-Derivate der Formel 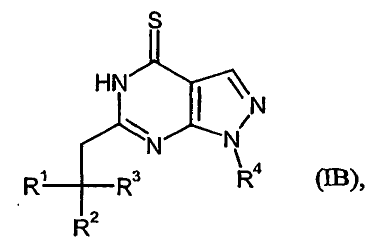
in welcher R¹, R², R³ und R⁴ die oben angegebenen Bedeutungen haben,
überführt,
und die resultierenden Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

7. Verbindungen nach einem der Ansprüche 1 bis 5 zur Behandlung und/oder Prophylaxe von Krankheiten.

8. Arzneimittel enthaltend mindestens eine der Verbindungen nach einem der Ansprüche 1 bis 5 und mindestens einen pharmazeutisch verträglichen, im wesentlichen nichtgiftigen Träger oder Exzipienten.

9. Verwendung der Verbindungen nach einem der Ansprüche 1 bis 5 zur Herstellung eines Arzneimittels zur Prophylaxe und/oder Behandlung von Störungen der Wahrnehmung, Konzentrationsleistung, Lern- und/oder Gedächtnisleistung.

10. Verwendung nach Anspruch 9, wobei die Störung eine Folge der Alzheimer'schen Krankheit ist.

11. Verwendung der Verbindungen nach einem der Ansprüche 1 bis 5 zur Herstellung eines Arzneimittels zur Verbesserung der Wahrnehmung, Konzentrationsleistung, Lern- und/oder Gedächtnisleistung.

## Claims

1. Compounds of the formula 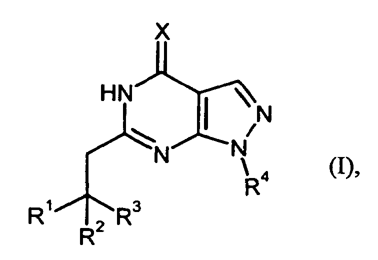
in which
R¹ is C₁-C₆-alkyl, trifluoromethyl, hydroxy, C₁-C₆-alkoxy, -C(=O)OR⁵ or -C(=O)NR⁶R⁷, where C₁-C₆-alkyl is optionally substituted by 1 to 3 radicals independently of one another selected from the group of hydroxy, C₁-C₆-alkoxy, halogen, trifluoromethyl, trifluoromethoxy, -C(=O)OR⁵ or -C(=O)NR⁶R⁷, and
R⁵ is C₁-C₆-alkyl,
R⁶ and R⁷ are independently of one another hydrogen, C₆-C₁₀-aryl, C₁-C₆-alkyl, or
together with the nitrogen atom to which they are bonded form a 4- to 10-membered heterocyclyl,
R² is hydrogen, C₁-C₆-alkyl, trifluoromethyl, C₁-C₆-alkoxy,
or
R¹ and R² together with the carbon atom to which they are bonded form C₃-C₈-cycloalkyl, C₃-C₈-cycloalkenyl or 4- to 10-membered heterocyclyl, which are optionally substituted by up to 2 substituents from the group of C₁-C₆-alkyl, C₁-C₆-alkoxy, hydroxy, oxo, -C(=O)OR⁸, and
R⁸ is C₁-C₆-alkyl or benzyl,
R³ is hydrogen or C₁-C₆-alkyl,
R⁴ is pentan-3-yl, C₃-C₆-cycloalkyl,
X is oxygen or sulphur,
and the salts, solvates and/or solvates of the salts thereof.

2. Compounds according to Claim 1, wherein
R¹ is C₁-C₆-alkyl, hydroxy, C₁-C₆-alkoxy, -C(=O)OR⁵ or -C(=O)NR⁶R⁷, where C₁-C₆-alkyl is optionally substituted by hydroxy, C₁-C₆-alkoxy, -C(=O)OR⁵ or -C(=O)NR⁶R⁷, and
R⁵ is C₁-C₆-alkyl,
R⁶ and R⁷ are independently of one another hydrogen, C₆-C₁₀-aryl, C₁-C₆-alkyl, or
together with the nitrogen atom to which they are bonded form a 4- to 10-membered heterocyclyl,
R² is hydrogen, C₁-C₆-alkyl, C₁-C₆-alkoxy,
or
R¹ and R² together with the carbon atom to which they are bonded form C₃-C₈-cycloalkyl, C₃-C₈-cycloalkenyl or 4- to 10-membered heterocyclyl, which are optionally substituted by up to 2 substituents from the group of C₁-C₆-alkyl, C₁-C₆-alkoxy, hydroxy, oxo, -C(=O)OR⁸, and
R⁸ is C₁-C₆-alkyl or benzyl,
R³ is hydrogen or C₁-C₆-alkyl,
R⁴ is pentan-3-yl, C₄-C₆-cycloalkyl,
X is oxygen or sulphur,
and the salts, solvates and/or solvates of the salts thereof.

3. Compounds according to Claims 1 and 2, wherein
R¹ is C₁-C₄-alkyl, hydroxy, C₁-C₄-alkoxy, -C(=O)OR⁵ or -C(=O)NR⁶R⁷, where C₁-C₄-alkyl is optionally substituted by hydroxy, trifluoromethyl, C₁-C₄-alkoxy, -C(=O)OR⁵ or -C(=O)NR⁶R⁷, and
R⁵ is C₁-C₄-alkyl,
R⁶ and R⁷ are independently of one another hydrogen, phenyl, C₁₋C₄-alkyl, or
together with the nitrogen atom to which they are bonded form a 5- to 6-membered heterocyclyl,
R² is hydrogen, C₁-C₄-alkyl, trifluoromethyl, C₁-C₄-alkoxy,
or
R¹ and R² together with the carbon atom to which they are bonded form C₅-C₆-cycloalkyl, C₅-C₆-cycloalkenyl or 5- to 6-membered heterocyclyl, which are optionally substituted by up to 2 substituents from the group of C₁-C₄-alkyl, C₁-C₄-alkoxy, hydroxy, oxo, -C(=O)OR⁸, and
R⁸ is C₁-C₄-alkyl or benzyl,
R³ is hydrogen,
R⁴ is pentan-3-yl, C₅-C₆-cycloalkyl,
X is oxygen or sulphur,
and the salts, solvates and/or solvates of the salts thereof.

4. Compounds according to Claims 1 to 3, wherein
R¹ is methyl, ethyl, isopropyl, trifluoromethyl, methoxycarbonyl, ethoxycarbonyl or -C(=O)NR⁶R⁷, where methyl is optionally substituted by methoxycarbonyl or ethoxycarbonyl, and
R⁶ is phenyl and
R⁷ is hydrogen,
R² is hydrogen, methyl, trifluoromethyl, or
R¹ and R² together with the carbon atom to which they are bonded form cyclopentyl, cyclohexyl, cyclopentenyl or tetrahydrofuryl, where cyclohexyl is optionally substituted by methyl, and
R³ is hydrogen,
R⁴ is pentan-3-yl, C₅-C₆-cycloalkyl,
X is oxygen or sulphur,
and the salts, solvates and/or solvates of the salts thereof.

5. Compounds according to Claims 1 to 4, wherein
R¹ is methyl, ethyl, isopropyl, methoxycarbonyl, ethoxycarbonyl or C(=O)NR⁶R⁷, where methyl is optionally substituted by methoxycarbonyl or ethoxycarbonyl, and
R⁶ is phenyl and
R⁷ is hydrogen,
R² is hydrogen, methyl, or
R¹ and R² together with the carbon atom to which they are bonded form cyclopentyl, cyclohexyl, cyclopentenyl or tetrahydrofuryl, where cyclohexyl is optionally substituted by methyl, and
R³ is hydrogen,
R⁴ is pentan-3-yl, C₅-C₆-cycloalkyl,
X is oxygen,
and the salts, solvates and/or solvates of the salts thereof.

6. Process for preparing compounds according to Claims 1 to 5, **characterized in that**
[A] compounds of the formula 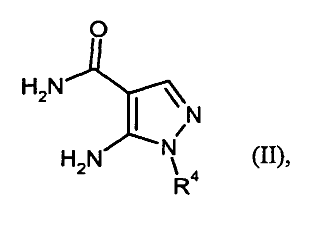
in which R⁴ has the meanings indicated above,
are converted by reaction with a compound of the formula 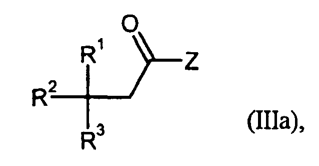
in which R¹, R² and R³ have the meanings indicated above,
and
Z is chlorine or bromine,
in an inert solvent and in the presence of a base initially into compounds of the formula 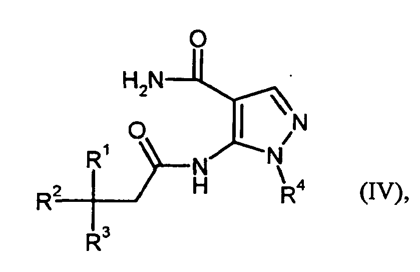
in which R¹, R², R³ and R⁴ have the meanings indicated above,
then cyclized in an inert solvent and in the presence of a base to compounds of the formula 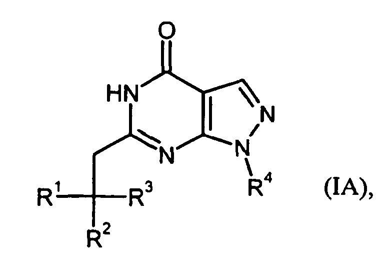
in which R¹, R², R³ and R⁴ have the meanings indicated above,
or
[B] compounds of the formula (II) are reacted, with direct cyclization to (IA), with a compound of the formula 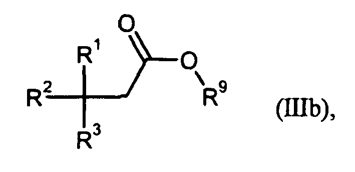
in which R¹, R² and R³ have the meanings indicated above
and
R⁹ is methyl or ethyl,
in an inert solvent and in the presence of a base,
or
[C] compounds of the formula 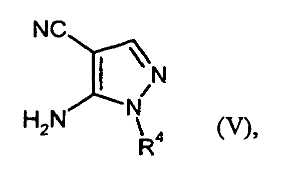
in which R⁴ has the meanings indicated above,
are initially converted by reaction with a compound of the formula (IIIa) in an inert solvent and in the presence of a base into compounds of the formula 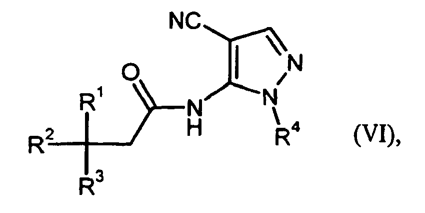
in which R¹, R², R³ and R⁴ have the meanings indicated above,
and the latter are cyclized in a second step in an inert solvent and in the presence of a base and of an oxidizing agent to (IA),
and the compounds of the formula (IA) are where appropriate then converted by reaction with a sulphurizing agent such as, for example, diphosphorus pentasulphide into the thiono derivatives of the formula 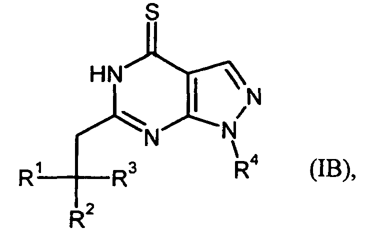
in which R¹, R², R³ and R⁴ have the meanings indicated above,
and the resulting compounds of the formula (I) are reacted where appropriate with the appropriate (i) solvents and/or (ii) bases or acids to give the solvates, salts and/or solvates of the salts thereof.

7. Compounds according to any of Claims 1 to 5 for the treatment and/or prophylaxis of diseases.

8. Medicament comprising at least one of the compounds according to any of Claims 1 to 5 and at least one pharmaceutically acceptable, essentially nontoxic carrier or excipients.

9. Use of the compounds according to any of Claims 1 to 5 for producing a medicament for the prophylaxis and/or treatment of impairments of perception, concentration, learning and/or memory.

10. Use according to Claim 9, wherein the impairment is a consequence of Alzheimer's disease.

11. Use of the compounds according to any of Claims 1 to 5 for producing a medicament for improving perception, concentration, learning and/or memory.

## Revendications

1. Composés de formule 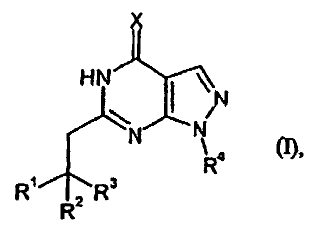 dans laquelle
R¹ est un reste alkyle en C₁ à C₆, trifluorométhyle, hydroxy, alkoxy en C₁ à C₆, -C(=O)OR⁵ ou -C(=O)NR⁶R⁷, le reste alkyle en C₁ à C₆ étant éventuellement substitué avec un à trois restes choisis, indépendamment les uns des autres, dans le groupe des restes hydroxy, alkoxy en C₁ à C₆, halogéno, trifluorométhyle, trifluorométhoxy, -C(=O)OR⁵ ou -C(=O)NR⁶R⁷, et
R⁵ est un reste alkyle en C₁ à C₆,
R⁶ et R⁷ représentent, indépendamment l'un de l'autre, l'hydrogène, un reste aryle en C₆ à C₁₀, alkyle en C₁ à C₆, ou bien ils forment conjointement avec l'atome d'azote auxquels ils sont liés un groupe hétérocyclyle tétragonal à décagonal,
R² représente l'hydrogène, un reste alkyle en C₁ à C₆, trifluorométhyle, alkoxy en C₁ à C₆,
ou bien
R¹ et R² forment, conjointement avec l'atome de carbone auquel ils sont liés, un reste cycloalkyle en C₃ à C₈, cycloalcényle en C₃ à C₈ ou hétérocyclyle tétragonal à décagonal, qui portent éventuellement jusqu'à deux substituants choisis dans le groupe des substituants alkyle en C₁ à C₆, alkoxy en C₁ à C₆, hydroxy, oxo, -C(=O)OR⁸, et
R⁸ est un reste alkyle en C₁ à C₆ ou benzyle,
R³ représente l'hydrogène ou un reste alkyle en C₁ à C₆,
R⁴ est un reste pentane-3-yle, cycloalkyle en C₃ à C₆,
X représente l'oxygène ou le soufre,
ainsi que leurs sels, leurs produits de solvatation et/ou les produits de solvatation de leurs sels.

2. Composés suivant la revendication 1, dans lesquels
R¹ est un reste alkyle en C₁ à C₆, hydroxy, alkoxy en C₁ à C₆, -C(=O)OR⁵ ou -C(=O)NR⁶R⁷, le reste alkyle en C₁ à C₆ portant éventuellement un substituant hydroxy, alkoxy en C₁ à C₆, -C(=O)OR⁵ ou -C(=O)NR⁶R⁷, et
R⁵ est un reste alkyle en C₁ à C₆,
R⁶ et R⁷ représentent, indépendamment l'un de l'autre, l'hydrogène, un reste aryle en C₆ à C₁₀ ou alkyle en C₁ à C₆, ou forment conjointement avec l'atome d'azote auxquels ils sont liés un reste hétérocyclyle tétragonal à décagonal,
R² représente l'hydrogène, un reste alkyle en C₁ à C₆, alkoxy en C₁ à C₆,
ou bien
R¹ et R² forment, conjointement avec l'atome de carbone auquel ils sont liés, un reste cycloalkyle en C₃ à C₈, cycloalcényle en C₃ à C₈ ou hétérocyclyle tétragonal à décagonal, qui portent éventuellement jusqu'à deux substituants du groupe des substituants alkyle en C₁ à C₆, alkoxy en C₁ à C₆, hydroxy, oxo, -C(=O)OR⁸, et
R⁸ est un reste alkyle en C₁ à C₆ ou benzyle,
R³ représente l'hydrogène ou un reste alkyle en C₁ à C₆,
R⁴ est un reste pentane-3-yle, cycloalkyle en C₄ à C₆,
X représente l'oxygène ou le soufre,
ainsi que leurs sels, leurs produits de solvatation et/ou les produits de solvatation de leurs sels.

3. Composés suivant les revendications 1 et 2, dans lesquels
R¹ est un reste alkyle en C₁ à C₄, hydroxy, alkoxy en C₁ à C₄, -C(=O)OR⁵ ou -C(=O)NR⁶R⁷, le reste alkyle en C₁ à C₄ portant éventuellement un substituant hydroxy, trifluorométhyle, alkoxy en C₁ à C₄, -C(=O)OR⁵ ou -C(=O)NR⁶R⁷, et
R⁵ est un reste alkyle en C₁ à C₄,
R⁶ et R⁷ représentent, indépendamment l'un de l'autre, l'hydrogène, un reste phényle, alkyle en C₁ à C₆, ou forment conjointement avec l'atome d'azote auxquels ils sont liés un groupe hétérocyclyle pentagonal ou hexagonal,
R² représente l'hydrogène, un reste alkyle en C₁ à C₄, trifluorométhyle, alkoxy en C₁ à C₄,
ou bien
R¹ et R² forment, conjointement avec l'atome de carbone auquel ils sont liés, un reste cycloalkyle en C₅ ou C₆, cycloalcényle en C₅ ou C₆ ou hétérocyclyle pentagonal ou hexagonal, qui portent éventuellement jusqu'à deux substituants du groupe des substituants alkyle en C₁ à C₄, alkoxy en C₁ à C₄, hydroxy, oxo, -C(=O)OR⁸, et
R⁸ est un reste alkyle en C₁ à C₄ ou benzyle,
R³ représente l'hydrogène,
R⁴ est un reste pentane-3-yle, cycloalkyle en C₅ ou C₆,
X représente l'oxygène ou le soufre,
ainsi que leurs sels, leurs produits de solvatation et/ou les produits de solvatation de leurs sels.

4. Composés suivant les revendications 1 à 3, dans lesquels
R¹ est un reste méthyle, éthyle, isopropyle, trifluorométhyle, méthoxycarbonyle, éthoxycarbonyle ou -C(=O)NR⁶R⁷, le reste méthyle portant éventuellement un substituant méthoxycarbonyle ou éthoxycarbonyle, et
R⁶ est un reste phényle, et
R⁷ représente l'hydrogène,
R² représente l'hydrogène, un reste méthyle, trifluorométhyle,
ou bien
R¹ et R² forment, conjointement avec l'atome de carbone auquel ils sont liés, un reste cyclopentyle, cyclohexyle, cyclopentényle ou tétrahydrofuryle, le reste cyclohexyle portant éventuellement un substituant méthyle, et
R³ représente l'hydrogène,
R⁴ est un reste pentane-3-yle, cycloalkyle en C₅ ou C₆,
X représente l'oxygène ou le soufre,
ainsi que leurs sels, leurs produits de solvatation et/ou les produits de solvatation de leurs sels.

5. Composés suivant les revendications 1 à 4, dans lesquels
R¹ est un reste méthyle, éthyle, isopropyle, méthoxycarbonyle, éthoxycarbonyle ou -C(=O)NR⁶R⁷, le reste méthyle portant éventuellement un substituant méthoxycarbonyle ou éthoxycarbonyle, et
R⁶ est un reste phényle, et
R⁷ représente l'hydrogène,
R² représente l'hydrogène, un reste méthyle,
ou bien
R¹ et R² forment, conjointement avec l'atome de carbone auquel ils sont liés, un reste cyclopentyle, cyclohexyle, cyclopentényle ou tétrahydrofuryle, le reste cyclohexyle portant éventuellement un substituant méthyle, et
R³ représente l'hydrogène,
R⁴ est un reste pentane-3-yle, cycloalkyle en C₅ ou C₆,
X représente l'oxygène,
ainsi que leurs sels, leurs produits de solvatation et/ou les produits de solvatation de leurs sels.

6. Procédé de production de composés suivant les revendications 1 à 5, **caractérisé en ce que** :
[A] on transforme tout d'abord des composés de formule 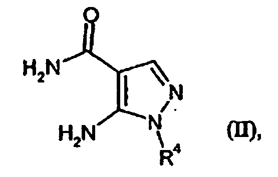
dans laquelle R⁴ a les définitions indiquées ci-dessus,
par réaction avec un composé de formule 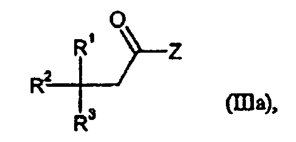
dans laquelle R¹, R² et R³ ont les définitions indiquées ci-dessus, et
Z représente le chlore ou le brome,
dans un solvant inerte et en présence d'une base, en composés de formule 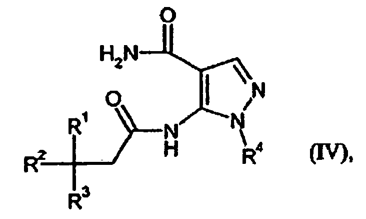
dans laquelle R¹, R², R³ et R⁴ ont les définitions indiquées ci-dessus,
qu'on cyclise ensuite dans un solvant inerte, en présence d'une base, en composés de formule 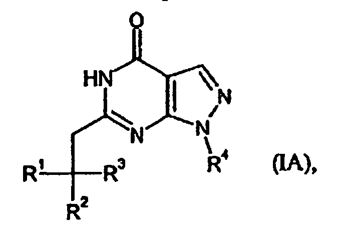
dans laquelle R¹, R², R³ et R⁴ ont les définitions indiquées ci-dessus,
ou bien
[B] on fait réagir des composés de formule (II), avec cyclisation directe en (IA) avec un composé de formule 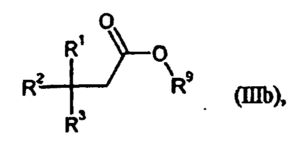
dans laquelle R¹, R² et R³ ont les définitions indiquées ci-dessus,
et
R⁹ est un reste méthyle ou éthyle,
dans un solvant inerte et en présence d'une base,
ou bien
[C] on transforme tout d'abord des composés de formule 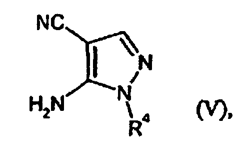
dans laquelle R⁴ a les définitions indiquées ci-dessus,
par réaction avec un composé de formule (IIIa) dans un solvant inerte et en présence d'une base, en composés de formule 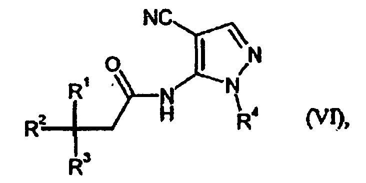
dans laquelle R¹, R², R³ et R⁴ ont les définitions indiquées ci-dessus,
qu'on cyclise dans une seconde étape en (IA) dans un solvant inerte et en présence d'une base et d'un agent oxydant,
puis on transforme éventuellement les composés de formule (IA), par réaction avec un agent de sulfuration tel que, par exemple, le pentasulfure de diphosphore, en les dérivés thiono de formule 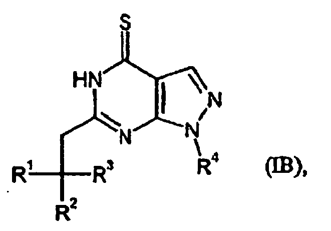
dans laquelle R¹, R², R³ et R⁴ ont les définitions indiquées ci-dessus,
et on fait réagir éventuellement les composés résultants de formule (I) avec (i) des solvants convenables et/ou (ii) des bases ou acides convenables pour former leurs produits de solvatation, leurs sels et/ou des produits de solvatation de leurs sels.

7. Composés suivant l'une des revendications 1 à 5, destinés au traitement et/ou à la prophylaxie de maladies.

8. Médicaments contenant au moins l'un des composés selon l'une des revendications 1 à 5 et au moins un support ou excipient principalement non toxique, acceptable du point de vue pharmaceutique.

9. Utilisation des composés suivant l'une des revendications 1 à 5 pour la préparation d'un médicament destiné à la prophylaxie et/ou au traitement de troubles de la perception, de la capacité de concentration, de la capacité d'apprentissage et/ou de la capacité de mémoire.

10. Utilisation suivant la revendication 9, le trouble étant une conséquence de la maladie d'Alzheimer.

11. Utilisation des composés suivant l'une des revendications 1 à 5 pour la préparation d'un médicament destiné à améliorer la perception, la capacité de concentration, la capacité d'apprentissage et/ou la capacité de mémoire.
